(19) Europäisches Patentamt
European Patent Office
Office européen des brevets

(11) **EP 1 534 324 B1**

(12) **EUROPEAN PATENT SPECIFICATION**

(45) Date of publication and mention
of the grant of the patent:
**16.01.2019 Bulletin 2019/03**

(51) Int Cl.:
*A61K 39/00* (2006.01)     *A61K 31/70* (2006.01)
*A61P 35/00* (2006.01)     *A61P 31/00* (2006.01)
*G01N 33/574* (2006.01)    *C07H 5/06* (2006.01)
*A61K 47/54* (2017.01)     *A61K 38/45* (2006.01)

(21) Application number: **03792440.4**

(22) Date of filing: **20.08.2003**

(86) International application number:
**PCT/FI2003/000615**

(87) International publication number:
**WO 2004/017810 (04.03.2004 Gazette 2004/10)**

(54) **TUMOR SPECIFIC OLIGOSACCHARIDE EPITOPES AND USE THEREOF**

TUMORSPEZIFISCHE OLIGOSACCHARID-EPITOPE UND IHRE VERWENDUNG

EPITOPES D'OLIGOSACCHARIDE SPECIFIQUES DE TUMEUR ET LEUR UTILISATION

(84) Designated Contracting States:
**AT BE BG CH CY CZ DE DK EE ES FI FR GB GR
HU IE IT LI LU MC NL PT RO SE SI SK TR**
Designated Extension States:
**LT LV**

(30) Priority: **20.08.2002 PCT/FI02/00681**

(43) Date of publication of application:
**01.06.2005 Bulletin 2005/22**

(60) Divisional application:
**10184228.4 / 2 322 209**

(73) Proprietor: **Glykos Finland Oy
00790 Helsinki (FI)**

(72) Inventors:
• **NATUNEN, Jari**
**FIN-01520 Vantaa (FI)**
• **TENEBERG, Susann**
**43063 Hindas (SE)**
• **KARLSSON, Karl-Anders**
**41143 Göteborg (SE)**
• **SATOMAA, Tero**
**00700 Helsinki (FI)**
• **HEISKANEN, Annamari**
**00370 Helsinki (FI)**

(74) Representative: **Seppo Laine Oy
Itämerenkatu 3 B
00180 Helsinki (FI)**

(56) References cited:
EP-A1- 0 255 342        EP-A1- 0 334 962
WO-A1-00/21552          WO-A1-91/14697
WO-A1-03/016915         WO-A1-03/016915
WO-A2-01/87321          WO-A2-03/094842
DE-A1- 3 807 594        US-A- 6 075 134

• MOCZAR E ET AL: "PREPARATION OF N ACETYL GLUCOSAMINE DERIVATIVES OF PROTEINS" FEBS LETTERS, vol. 50, no. 3, 1975, pages 300-302, XP002411483 ISSN: 0014-5793
• ZENG Y ET AL: "Synthesis of aryl azide derivatives of UDP-GlcNAc and UDP-GalNAc and their use for the affinity labeling of glycosyltransferases and the UDP- HexNAc pyrophosphorylase" ANALYTICAL BIOCHEMISTRY 1996 UNITED STATES, vol. 239, no. 1, 1996, pages 99-106, XP002411484 ISSN: 0003-2697
• JOHNSON P.J. ET AL.: 'Structures of disease-specific serum alpha-fetoprotein isoforms' BRITISH JOURNAL OF CANCER vol. 83, no. 10, 2000, pages 1330 - 1337, XP002960522
• JOHNSON P.J. ET AL.: 'Glycan composition of serum alpha-fetoprotein in patients with hepatocellular carcinoma and non-seminomatous germ cell tumour' BRITISH JOURNAL OF CANCER vol. 81, no. 7, 1999, pages 1188 - 1195, XP001032486

- DENNIS JAMES W. ET AL.: 'Asn-linked oligosaccharides in lectin-resistant tumor-cell mutants with varying metastatic potential' EUR. J. BIOCHEM. vol. 161, 1986, pages 359 - 373, XP002960523
- KATSUKO YAMASHITA ET AL.: 'Comparative study of the sugar chains of gamma-glutamyltranspeptidases purified from rat liver and rat AH-66 hepatoma cells' CANCER RESEARCH vol. 43, November 1983, pages 5059 - 5063, XP002970538
- KATSUKO YAMASHITA ET AL.: 'Structural study of the sugar chains of alpha-amylases produced ectopically in tumors' J. BIOCHEM. vol. 90, 1981, pages 1281 - 1289, XP002985187
- CHECHIK BORIS E. ET AL.: 'Increased expression of highly branched N-linked oligosaccharides terminating in N-acetylglucosamine residues in neoplastic and sclerodermal chicken fibroblasts' HISTOCHEMICAL JOURNAL vol. 24, 1992, pages 15 - 20, XP002960524
- VOLMAN GAIL NIXON ET AL.: 'Analysis of asparagine-linked oligosaccharide structures of chronic lymphocytic leukemia cells' MOLECULAR IMMUNOLOGY vol. 24, no. 8, 1989, pages 871 - 886, XP002960525
- TAMAO ENDO ET AL.: 'Comparative study of the sugar chains of alkaline phosphatases purified from rat liver and rat AH-130 hepatoma cells occurrence of fucosylated high-mannose-type and hybrid-type sugar chains' EUR. J. BIOCHEM. vol. 236, 1996, pages 579 - 590, XP002960521
- ABDELHADI REBBAA ET AL.: 'Expression of bisecting G1cNac in pediatric brain tumors and its association with tumor cell response to vinblastine' CLINICAL CANCER RESEARCH vol. 5, 1999, pages 3661 - 3668, XP002960529
- NOBUHISA OHTANI ET AL.: 'Abnormality of the oligosaccharide moiety of human immunoglobulin G in sera of patients with either rheumatoid arthritis or cancer' JPN. J. ELECTROPH. vol. 46, no. 163, 2002, pages 163 - 167, XP008046424
- MEICHENIN MARC ET AL.: 'Tk, a new colon tumor-associated antigen resulting from altered O-glycosylation 1' CANCER RESEARCH vol. 60, 01 October 2000, pages 5499 - 5507, XP002960527
- HANISCH F.-G. ET AL.: 'Monoclonal antibody 2B5 defines a truncated O-glycan, GlcNAcbeta 1-3Galbeta 1 4GlcNacbeta 1-6 (GalNAc), on mucins from deep gastric and duodenal glands as well as metaplasia and neoplasia of gastric differentiation' CANCER RESEARCH vol. 53, 15 October 1993, pages 4791 - 4796, XP002960526
- JUN NAKAYAMA ET AL.: 'Griffonia simplicifolia agglutinin-2-binding glycoprotein as a novel carbohydrate antigen of human colonic carcinoma' JPN. J. CANCER RES. vol. 81, April 1990, pages 388 - 395, XP002970539
- DATABASE WPI Week 199049, Derwent Publications Ltd., London, GB; Class B04, AN 1990-365935, XP002974358 & JP 2 264 864 A (SHINETSU CHEM IND CO LTD) 29 October 1990
- JIE HU ET AL.: 'Structural characterization of intermediates in the biosynthetic pathway of neolacto glycosphingolipids: differential expression in human leukaemia cells' GLYCOBIOLOGY vol. 4, no. 3, 1994, pages 251 - 257, XP002970541
- HOLMES ERIC H. ET AL.: 'Isolation and fine-structure characterization of four monoclonal antibodies reactive with glycoconjugates containing terminal GlcNAc residues: application to aspects of lacto-series tumor antigen biosynthesis 1' ARCHIVES OF BIOCHEMISTRY AND BIOPHYSICS vol. 288, no. 1, July 1991, pages 87 - 96, XP002961528
- PATENT ABSTRACTS OF JAPAN vol. 2000, no. 10 17 November 2000 & JP 2000 191685 A (NIPPON KOUTAI KENKYUSHO:KK) 11 July 2000
- BUELTER THOMAS ET AL.: 'Chemoenzymatic synthesis of biotinylated nucleotide sugars as substrates for glycosyltransferases' CHEMBIOCHEM vol. 2, 2001, pages 884 - 894, XP002974357
- ZHAO CHUN CHEN ET AL.: 'Synthesis of alpha-Gal epitopes (Galalpha 1-3Galbeta 1-4GlcNAc-R) on human tumor cells by recombinant alpha 1,3galactosyltransferase produced in Pichia pastoris' GLYCOBIOLOGY vol. 11, no. 7, 2001, pages 577 - 586, XP002974356
- HENION TIMOTHY R. ET AL.: 'Synthesis of a-Gal epitopes on influenza virus vaccines, by recombinant alpha 1,3galactosyltransferase, enables the formation of immune complexes with the natural anti-Gal antibody' VACCINE vol. 15, no. 11, 1997, pages 1174 - 1182, XP004086586
- REIKO SADAMOTO ET AL.: 'Cell wall engineering of living bacteria through biosynthesis' METHODS IN ENZYMOLOGY vol. 362, 2003, pages 273 - 286, XP002974355
- LEMIEUX GEORGE A. ET AL.: 'Modulating cell surface immunoreactivity by metabolic induction of unnatural carbohydrate antigens' CHEMISTRY & BIOLOGY vol. 8, 2001, pages 265 - 275, XP002202102
- SAXON ELIANA ET AL.: 'Chemical and biological strategies for engineering cell surface glycosylation' ANNU. REV. CELL DEV. BIOL. vol. 17, 2001, pages 1 - 23, XP002974354
- BJLTER THOMAS ET AL: "Chemoenzymatic synthesis of biotinylated nucleotide sugars as substrates for glycosyltransferases", CHEMBIOCHEM - A EUROPEAN JOURNAL OF CHEMICAL BIOLOGY, WILEY VCH, WEINHEIM, DE, vol. 2, 1 January 2001 (2001-01-01), pages 884-894, XP002974357, ISSN: 1439-4227, DOI: 10.1002/1439-7633(20011203)2:12<884::AID-CBIC884>3.0.CO;2-2

**Description**

FIELD OF THE INVENTION

The present description relates to oligosaccharide sequences, which are specifically expressed by human tumors. The invention is also related to diagnostics of cancer and malignancies.

BACKGROUND OF THE INVENTION

Various tumors express oligosaccharide sequences which are different from the non-malignant glycosylation of the same cell or tissue type. Examples of the known or speculated cancer associated oligosaccharide structures include: glycolipid structures such as globo-H (Fucα2Galβ3GalNAcβ3Galα4LacβCer), gangliosides: GM1 Galβ3GalNAcβ4(NeuNAcα3)LacβCer or GD2 GalNAcβ4(NeuNAcα8NeuNAcα3)LacβCer; Lewis-type fucosylated structures such as Lewis a and x: Galβ3/4(Fucα4/3)GlcNAc, Lewis y: Fucα2Galβ4(Fucα3)GlcNAc, sialyl-Lewis x: NeuNAcα3Galβ4(Fucα3)GlcNAc, and some combinations of these on polylactosamine chains; O-glycan core structures, such as T-antigen Galβ3GalNAcαSer/Thr-Protein, Tn-antigen GalNAcαSer/Thr-Protein or sialyl Tn-antigen NeuNAcα6GalNAcαSer/Thr-Protein. Presence of non-human structures such as N-glycolyl-neuraminic acid in cancers has also been indicated. Association and specificity of oligosaccharide structures with regard to cancers have been well established only in few cases, some of the structures are present in normal cells and tissues and are possibly only more concentrated in cancers.

One report has indicated that structures with terminal GlcNAcβ3Galβ4GlcNAc sequence are present in human leukaemia cells (Hu et al., 1994). The structures may also be equally present on normal leukocytes. Thus, the relation of the finding to glycosylation patterns generally present in solid tumors was not indicated. This type of saccharide structures may be a part of rare normal glycosylations of human tissues: GlcNAcβ3Galβ4GlcNAcβ6 sequence linked on O-glycans is probably present on human gastric mucin. A study shows that a monoclonal antibody recognizing GlcNAcβ3Galβ4GlcNAcβ6 sequence may possibly recognize similar structures on malignant tissues, such as mucinous ovarian neoplasms, pseudopyloric metaplasia of gallbladder and pancreatic epithelia, gastric differentiated carcinoma of stomach, gallbladder and pancreas, and on non-malignant tissues, such as human amniotic fluid, but, however, the structures from malignat tissues were not characterized (Hanisch et al., 1993). The antibody did not recognize neoglycolipid structure GlcNAcβ3Galβ4GlcNAcβ3Galβ4 nor carcinomas of lung, colorectum, endometrium or other organs. Another monoclonal antibody raised against testicular cells probably recognizes branched N-acetyllactosamines such as GlcNAcβ3(GlcNAcβ6)Galβ4GlcNAc-(Symington et al., 1984). Terminal GlcNAc has also been reported from mucins of human foetal mucin (Hounsell et al., 1989). In normal tissues terminal GlcNAc may be present in minor amounts as biosynthetic intermediates in the biosynthesis of poly-N-acetyllactosamines.

Several monoclonal antibodies has been raised against a semisynthetic glycolipid GlcNAcβ3Galβ4GlcNAcβ3LacβCer, these antibodies were shown to recognize glycolipids from cultured colon cancer cell lines and tumors (Holmes et al., 1991). However, the antibodies recognized several structures and the binding data was contradictory. Moreover the glycolipids were not recognized by all of the antibodies and the glycolipid structures from cancer cells or tumors were not characterized. Therefore the presence of terminal GlcNAc structures on tumors were not established. Another study showed production of a monoclonal antibody against GlcNAcβ3LacβCer (Nakamura et al., 1993). This antibody also weakly recognized the pentasaccharide structure described above. Moreover, the antibody recognized a protease sensitive epitope on COS-1 cells, which cell line is not of human origin. The immunization protocols of these studies did not describe induced antibody responses against polyvalent conjugates of the saccharides, but immunization by glycolipids.

Normally there are large amounts of antibodies recognizing terminal GlcNAc structures in human serum. There are also a class of natural antibodies recognizing terminal Galα3Galβ4GlcNAc- structures. The Galα antigen is not naturally present in man and recently it was also shown that the natural antibodies bind structures such as GalNAcα3Galβ4GlcNAc, GalNAcβ3Galβ4GlcNAc, and GlcNAcβ3Galβ4GlcNAc (Teneberg et al., 1996). The X2-structure, GalNAcβ3Galβ4GlcNAc, is a normal antigen on human tissues and structures GalNAcα3Galβ4GlcNAc and Galα3Galβ4GlcNAc have not been described from normal or cancer tissues. Thus, the present finding that the terminal GlcNAc structure is a tumor antigen indicates that the actual function of the natural antibodies might be the prevention of cancers having terminal GlcNAc structures.

The following patents describe cancer antigens and their use for making antibodies for therapeutic and diagnostic uses and for cancer vaccines. The antigen structures are not related to saccharides described in the present description:

Cancer vaccines: US patent Nos. 5,102,663; 5,660,834; 5,747,048; 5,229,289 and 6,083,929.

Therapeutic antibodies: US patent Nos. 4,851,511; 4,904,596; 5,874,060; 6,025,481 and 5,795,961.

Diagnostics: US patent Nos. 4,725,557; 5,059,520; 5,171,667; 5,173,292; 6,090,789; 5,708,163; 5,679,769; 5,543,505; 5,902,725 and 6,203,999.

In the prior art tumor diagnostic and therapheutic antibodies recognizing chitobiosemannose trisaccharides has been described in DE 38 07 594 A1. The application also describes other N-glycans with numerous varying terminal structures some of which may comprise also non-reducing terminal N-acetyl glucosamine. Several of the desired structures have been characterized as normal glycans and not cancer specific structures. The application claims to describe structures useful for cancer applications. Hwever, it is not clear from the invention what the structure of the desired glycan is. Formel (I) may indicate presence of non-reducing terminal GlcNAc, if it is unconventionally read from right to left. However the Formel (I) does not indicate the linkage structure of the terminal GlcNAc. The Formel (III) indicates that the GlcNAc residues are $\alpha2$, $\alpha4$, or $\alpha6$-linked. The present description is not directed to such unusual structures. The present description is directed to human tumor specific glycans_comprising non-reducing end terminal $\beta$-linked GlcNAc residues.

Patent application WO 00/21552 claims several unusual O-glycan structures isolated from bovine submaxillary mucin. Two of the structures such as GlcNAc$\beta$6GalNAc$\alpha$6GalNAc and GalNAc$\beta$3(GlcNAc$\beta$6)GalNAc comprise terminal GlcNAc-residues. The application did not indicate that said structures would also be related to bovine or human cancers. The present description is not directed to these structures comprising two GalNAc-residues. The application contains speculation about potential therapheutic use of the structures as antigens related to cancer. However, it has not been shown that the structures are related to bovine cancer when these are present in bovine normal submaxillary secretion. Moreover, it is even less probable that the structures would be present in human tissues, the glycosylations are species specific and vary between human and bovine, e.g. bovine and human glycosyltransferase and glycosylation profiles are different. The human genome is also known and thus gylcosyltransferases which could be related to synthesis of the claimed bovine structures should have been now produced and characterized from human. So far none of these has been described in human, or human cancer.

Meichenin et al, 2000 shows a murine monoclonal antibody which can bind to some oligosaccharides containing terminal GlcNAc$\beta$ and to certain human tissue cancer samples. The antibody was produced in mouse using the endo-beta-galactosidase treated red cells. However, the article does not establish any human specific immunotherapy with a specific antibody or other carbohydrate specific binding substance binding to an immunogenic carbohydrate. Moreover, the fact that such an antibody with limited specificity was formed in mouse does not indicate that similar antibody would be formed in human, or it would be tolerable in human, or be useful in context of individual human tumors of specific kind, since immune reactions to carbohydrates are species specific.

Endo et al, 1996 describes N-glycan type oligosaccharides from soluble protein alkaline phosphatase of rat hepatoma AH-130 cell line. Again, material of animal origin is speculated in view of cancer specificity. Therefore, no diagnostics or therapy of human cancer is disclosed.

Patent application FI20011671 described the general useability of terminal GlcNAc-structures in tumor therapy. The application described specific polylactosamine type oligosaccharide sequences containing terminal GlcNAc linked to Gal especially found from glycolipid. The application indicated that the structures can be part of N-linked glycan or O-linked glycans, and that the tumorspecific oligosaccharide sequences can also be linked to O-glycosidic GalNAc. The application did not disclose exact structures of the O-linked or N-linked glycans

The present application also show for the first time the useability of the oligosaccharide sequences of the terminal beta-GlcNAc sequences for cancers of lung, stomach, colon, larynx and mucinous carcinomas, especially mucinous ovarian carcinomas forming a group of epithlial type and/or mucin secreting cancers. The present description is further especially directed to human cancer specific protein linked GlcNAc$\beta$-structures. The present description is also further especially directed to the specified N-glycans and O-glycans and protein linked GlcNAc. The preferred structures form a specific family of terminal specifically "protein linked GlcNAc$\beta$-structures" which are human protein linked defective glycosylation present in human cancer. Particularly, the preferred terminal beta-GlcNAc O-glycans and N-glycans form a specific family of human cancer specific "protein linked GlcNAc$\beta$-glycan cores" which are result of defective galactosylation of cancer or tumor tissue.

The present application further describes human natural antibodies and more specifically human cancer associated antibodies specifically recognizing preferred human terminal beta-linked GlcNAc structures. The present description also describes an enzyme based targeting of the cancer antigens by transferring a modified monosaccharide derivative on cancer cells.

Current therapies for cancer and numerous infectious diseases are not effective enough. Cancers are major cause of deaths in industrialized countries and devastating infectious diseases kill children and adults especially in developing countries. Infections are probably behind numerous life-style and other diseases of the industrialized world, like gastric ulcers caused by *Helicobacter pylori*.

Previous *in vitro* studies have described the transfer of sialyl-Lewis x-oligosaccharides on surface of a cultured cell type. The cells were used to study the binding of human selectins to the sialyl- Lewis x oligosaccharides. Similarily, a blood group B-antigen has been transferred to human erythrocytes, and it was shown to be recognized by anti-blood group B antibodies. Because of the unnatural structure of the GDP-Fuc(-B-antigen), the authors stated that the structure was unsuitable for *in vivo* use. According to present description, it is possible to use the unnatural structures as modified monosaccharides to target microbial pathogens, viruses, tumors or cancers.

A galactosyltransferase has been used to label human endo-beta-galactosidase modified erythrocytes (Viitala and Finne, 1984) and mouse teratocarcinoma cells (Spillmann and Finne, 1994) under *in vitro* conditions. Cell surface galactosyltransferases has been also studied in connection of various biological conditions. These studies do not describe diagnostics or therapy for any disease. The carbohydrates transferred are not monosaccharide conjugates according to the present description. The old studies utilized radioactively labeled UDP-Gal, the chemical structure of the Gal-residue is not changed but it contains atoms enriched with $^{14}$C or $^{3}$H.

A fluorecently labelled muramic acid has been transferred on bacteria and the method was speculated to be used to study vaccination against the compound transferred or for interaction studies between bacteria, the transfer reaction was not specific for a type of a bacterium. The method was aimed to be used in vitro and the cells had to be permeabilized to achieve very weak reactions by the peptidoglycan precursor molecules (Sadamoto, R. et al 2001). In contrast the transfer reactions described by the present description for bacteria are targeted to transfer of the carbohydrates directly to the cell surface.

Moreover the previous technology describes *in vitro* transfer of Gal on acceptors of a cancer cell by $\alpha$1-3galactosyltransferase. The monosaccharide to be transferred is not modified nor aimed for blocking of a pathogenesis-inducing carbohydrate receptor. The invention does not describe transfer of immunologically active or toxic monosaccharide conjugates, but the terminal structure formed is reactive to anti Gal$\alpha$1-3Gal-antibodies. The epitope was aimed for increasing immununoreactivity of cancer cells to be injected as cancer vaccine.

Previous art also described transfer of 6-biotinylated Gal to GlcNAc-BSA or hen egg white ovalbumin or transfer of fluoresceinyl-NeuNAc to glycoproteins of golgi apparathus. The studies did not describe the diagnostics according to the present description (Bulter T. et al. 2001).

Various cell types express oligosaccharide sequences which are different from the non-malignant glycosylation of the same cell or tissue type. Association and specificity of oligosaccharide structures with regard to cancers have been well established only in few cases, some of the structures are present in normal cells and tissues and are possibly only more concentrated in cancers. The present patent application also describes terminal N-acetylglucosamine containing tumor specific oligosaccharide sequences.

Normally there are large amounts of antibodies recognizing terminal GlcNAc structures in human serum. Thus, the previous finding that the terminal GlcNAc structure is a tumor antigen indicates that the actual function of the natural antibodies might be the prevention of cancers having terminal GlcNAc structures.

WO 03/094842 discloses conjugates of glucosamine and a central nervous system drug. The conjugates may be considered as suitable glycosyltransferase substrates.

Moczar et al., 1975, (FEBS Letters, 50(3):300-302) disclose polypeptide-N-acetylglucosamine conjugates used as substrates for glycosyltransferases and in immunological studies.

Zeng et al., 1996, (Analytical Biochemistry, 239(1):99-106) disclose aryl azide derivatives of UDP-GlcNAc and UDP-GalNAc as being substrates for glycosyltransferases.

Bülter et al., 2001, (Chembiochem - A European Journal of Chemical Biology, 2:884-894) disclose 6-modified saccharide compounds such as biotinylated nucleotide sugars as substrates for glyco syltransferases.

## SUMMARY OF THE INVENTION

The present invention is defined by the appended claims.

## BRIEF DESCRIPTION OF THE DRAWINGS

**Figure 1.** An autoradiogram of a thin-layer assay after overlayering and binding of GlcNAc$\beta$-specific *E. coli* bacterium demonstrating the tumor specificity of the oligosaccharide sequences containing terminal GlcNAc residue: non-acidic glycosphingolipids from hypernephroma tumor (first lane) and corresponding glycosphingolipid fraction from normal kidney (second lane).

**Figure 2.** Thin-layer overlay assays A) using [$^{35}$S]-labelled, GlcNAc$\beta$-specific *E. coli* and B) [$^{121}$I]-labelled Gal$\beta$4GlcNAc$\beta$-specific lectin from *Erythrina cristagalli.* Lanes 1-8: Subfractions of non-acid glycosphingolipids from human hypernephroma. Lane 9: Reference glycosphingolipid GlcNAcβ3Galβ4Glcβ1Cer. Lane 10: Reference glycosphingolipid globoside GalNAcβ3Galα4Galβ4Glcβ1Cer.

**Figure 3A.** Positive ion reflector mode MALDI-TOF mass spectrum of lung adenocarcinoma sample neutral glycans.

**Figure 3B.** Positive ion reflector mode MALDI-TOF mass spectrum of healthy lung sample neutral glycans.

**Figure 3C.** Positive ion reflector mode MALDI-TOF mass spectrum of lung adenocarcinoma sample neutral glycans after *S. pneumoniae* β-N-acetylglucosaminidase digestion.

**Figure 3D.** Positive ion reflector mode MALDI-TOF mass spectrum of lung adenocarcinoma neutral glycans after *S. pneumoniae* β-N-acetylglucosaminidase and jack bean α-mannosidase digestions.

**Figure 4A.** Negative ion linear mode MALDI-TOF mass spectrum of purified nucleotide sugars after UDP-galactosamine synthesis reaction.

**Figure 4B.** Negative ion linear mode MALDI-TOF mass spectrum of purified nucleotide sugars after the UDP-GalN-biotin synthesis reaction.

**Figure 5.** Structure of UDP-GalN-biotin, uridine 5'-diphospho-N-(6-biotinamidohexanoyl)galactosamine.

**Figure** 6. Autoradiography of [$^{14}$C]Gal labelled lung adenocarcinoma (a.) and healthy lung tissue (b.) sections.

**Figure 7A.** [$^{14}$C]Gal labelled oligosaccharides from N-glycosidase F digested lung adenocarcinoma sample. Glycans were subjected to gel filtration HPLC with a Superdex Peptide HR 10/30 column (Pharmacia, Sweden) in 50 mM $NH_4HCO_3$ (pH about 8.3) at a flow rate of 1 ml/min. 1 ml fractions were collected and counted for radioactivity. Fractions at 12 - 15 min were pooled.

**Figure 7B.** [$^{14}$C]Gal labelled oligosaccharides from N-glycosidase F digested lung adenocarcinoma sample. The 12 - 15 min pool from Superdex Peptide gel filtration HPLC (Fig. 10A) was subjected to HPLC with a 4.6x250 mm Hypercarb 5u column (Thermo Hypersil, USA) in 10 mM $NH_3$ at a flow rate of 0.7 ml/min, with a linear gradient of 0 % to 40 % acetonitrile in the mobile phase in 100 minutes. 0.7 ml fractions were collected and counted for radioactivity.

**Figure 7C.** [$^{14}$C]Gal labelled material released by nonreductive β-elimination from lung adenocarcinoma sample. The material was subjected to gel filtration HPLC with a Superdex Peptide HR 10/30 column (Pharmacia, Sweden) in 50 mM $NH_4HCO_3$ at a flow rate of 1 ml/min. 1 ml fractions were collected and counted for radioactivity. Fractions at 8 - 15 min (pool 1) were pooled as well as at 15 - 18 min (pool 2).

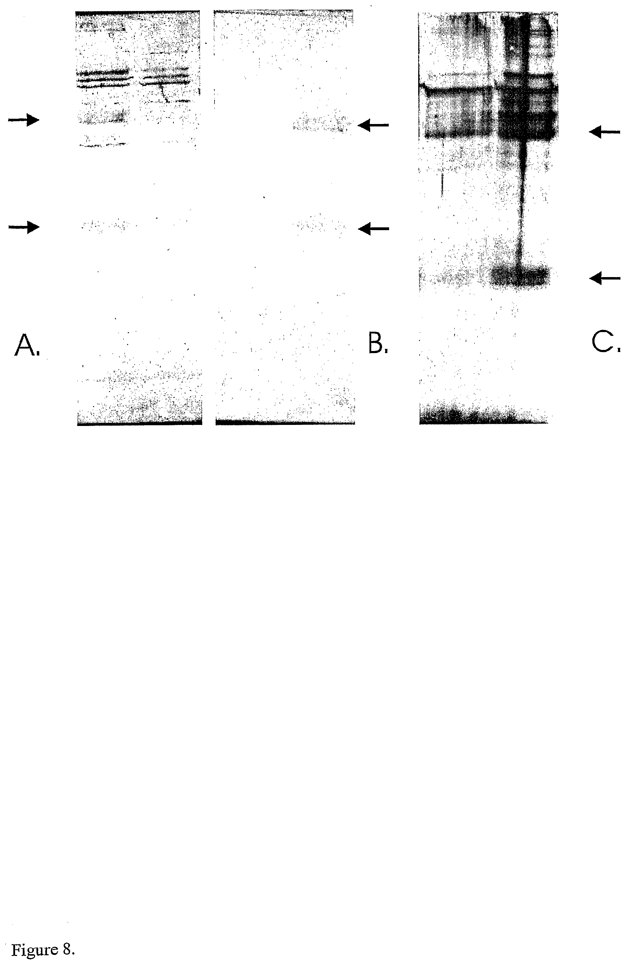

**Figure 8.** Coomassie Blue stained reducing SDS-PAGE gels. Arrowheads indicate the positions of IgG heavy and light chains, respectively. (A.) Serum from a person who had recovered from mucinous ovarian adenocarcinoma, GlcNAcβ1-6(Galβ1-3)GalNAcα Sepharose; left: 0.5 M GlcNAc elution, right: acidic elution. (B.) IgG from pooled human sera, GlcNAcβ1-6(Galβ1-3)GalNAcα Sepharose; left: 0.5 M GlcNAc elution, right: acidic elution. (C.) Silver stained gel. Serum from a person who had recovered from mucinous ovarian adenocarcinoma, Galβ1-4GlcNAcβ1-6(Galβ1-3)GalNAcα Sepharose; left: 0.5 M GlcNAc elution, right: acidic elution.

**Figure 9.** The chemical structure of UDP-GalN-PEG-fluorescein reagent.

**Figure 10.** Fluorescence microscopy of GalN-PEG-fluorescein labeled tumor tissue sections. A Control reaction without the enzyme at 460-490 nm. B. Control reaction without the enzyme at 530-550 nm. C. Reaction with the enzyme at 460-490 nm. D. Reaction with the enzyme at 530-550 nm. E. Reaction with the enzyme at 460-490 nm. F. Reaction with the enzyme at 530-550 nm.

## DETAILED DESCRIPTION OF THE INVENTION

The present description is directed to terminal β-linked N-acetylglucosamine oligosaccharide chain structures and protein linked monosaccharide N-acetylglucosamine which are presented by human tumors. The present description realizes specific defects in human tumors, which leads to cell surface and extracellular presentation of unsual carbohydrates comprising terminal N-acetylglucosamines. In general the terminal GlcNAc-structures are very rare in human normal tissues. The expression of the structures are caused by two factors. For the first the biosynthesis and degradation machinery in the tumor cells does not work properly. Many terminal structures in normal tissues comprise galactose on GlcNAc-residues, these structures are capped by sialic acids, blood group antigens and the like. The present data indicates that the cancer or tumor carbohydrates are exposed to unusual glycosidase activities and the defective glycosylation reactions by β4- and possibly also by β3-galactosyltransferases. Defects were observable in all 3 types of β-galactosylated oligosaccharide sequences. Secondly, the intracellular targeting and quality control of glycosylation including glycoprotein and glycolipid structures seem to be defective. In normal cells the glycosylation is under quality control which recirculates underglycosylated proteins and glycolipids to complete the natural glycosylation, so that in normal cells the amount of the structures disclosed in the present description are very rare on cell surfaces. In normal cells or tissues the oligosaccharide structures described by the present description are present in low amounts in human golgi apprathus, the known protein linked GlcNAc is considered to be almost exclusively a cytoplasmic nuclear protein modification. The defects in organization of the golgi apparathus leads to partial cell surface expression of the tumor specific structures described by the present description. Even intracellularily overexpressed structures according to the present description are directly useful for diagnostic applications.

The defects in galactosylation and presence of unusual glycosidase activities and loss of intracellular quality control lead to three types of tumor associated glycosylations:

    1. incomplete, undergalactosylated protein linked N-glycan and incomplete, undergalactosylated
    2. O-glycan core structures,
    3. moreover polylactosamines are also undergalactosylated as exemplified by poly-N-acetyllactosamine type glycolipid from human hypernephroma.
    These defects lead to several unusual terminal epitopes on glycoproteins. The present description is directed to the three groups of oligosaccharide epitopes. The current description notices for the first time similar general defect on all three types of glycan chains carrying normally β-galactosylated oligosaccharide sequences. Terminal β-linked GlcNAc is present as terminal structure. The structures indicate defects in enzymatic steps directly modifying the terminal GlcNAc-residues including β1,4(3)-Gal-transferase reactions and on the other hand increased glycosidase activities in the Golgi-pathway which could degradate terminal structures. However, the generality of the defect in 3 types of carbohydrates would indicate that organization of the golgi apparathus is so disturbed that terminally modifying enzymes located in late golgi cannot effectively modify all glycans expressed by tumor cells.
    Furthermore present description is directed to
    4. missdirected expression of protein linked N-acetylglucosamine monosaccharide on cancers and tumors.

The protein linked N-acetylglucosamine is in general present in so called O-GlcNAc-structures intracellularily. The present description notices strong overexpression of the protein linked N-acetylglucosamine. The overexpression leads to even some cell surface associated labeling of the O-linked GlcNAc on human tumors.

Though the terminal β-linked GlcNAc is common to the all three groups 1-3 above, it is realized that the subterminal structures have effect on the structures which should be recognized in diagnostic or theraphy of tumors or cancers. The present description is specifically directed to diagnostic uses of terminal oligosaccharide sequences comprising the terminal GlcNAc and one to several neighboring monosaccharide residues in the three defective oligosaccharide sequence groups. The present description is directed to the oligosaccharide sequences comprising nonreducing end terminal β-GlcNAc oligosaccharide sequences on polylactosamines, or O-glycans or N-glycans. The present description is directed to all of the defective glycosylation types when an analysis of normal glycosylation and potential general glycosylation defect is used together. The present description is further directed to the use of combinations of at least two of the four terminal GlcNAc type structures, especially the use of the polylactosamine type terminal GlcNAc-sequence in combination with the other terminal GlcNAc-structures as diagnostical targets.

General galactosylation defect and specific protein galactosylation defect

The present inventorsdiscovered that the defect of the glycosylation may be general and affect at least two classes of the four defective classes of terminal GlcNAcβ-glycosylations disclosed in the present description: O-glycans, N-glycans, polylactosamines and protein linked GlcNAc. The general defect of galactosylation may affect both specific protein linked glycosylations and glycolipid linked glycosylations, especially polylactosamine type terminal GlcNAc-structures of glycolipids. The present description is specifically directed to detecting presence of general galactosylation defect from a cancer sample and/or from normal tissue sample of a person. The present description is directed to the analysis of the general glycosylation defect producing the multiple types of terminal GlcNAc structures. The defect may be analyzed by an analysis of the carbohydrate samples as described by the present description. The galactosylation defect may be analyzed also by, for example, genetic or biochemical or cell biological means by analyzing production and/or activity and/or localization of enzymes or other proteins or biochemical mediators capable of producing the general glycosylation defect. The present description is directed to detecting general glycosylation defect when the intracellular structures involving glycan biosynthesis and/or degradation are defective. The present description is especially directed to the analysis of the general glycosylation defect by genetic and/or biochemical and/or cell biological means when the method is verified with the analysis of carbohydrate structures, preferably the carbohydrate structures are analyzed by mass spectrometry and/or with known chemical and/or enzymatic methods and/or by substances according to the present description specifically binding and/or modifying the terminal GlcNAcβ-structures disclosed in the present description.

The analysis of the general glycosylation defect in normal tissue(s) is according to the present description directed to be also used for predicting susceptibility or risk for a person for getting cancer and/or selecting persons for additional screening for cancer. The individual analysis of the normal tissue glycosylations and/or the general galactosylation defect disclosed in the present description is preferable as the normal glycosylation was noticed to vary in some cancer patients as described in the examples. The present description is also directed to the prediction of a high risk for cancer, especially for a person with "cancer type" terminal GlcNAc structures present in high amounts in normal tissue.

Specific glycosylation defect, especially protein glycosylation defect

The present description further discloses a glycosylation defect wherein the intracellular structures involving glycan biosynthesis and/or degradation are defective. When a specific class of terminal GlcNAcβ-structures is defective, the specific glycosylation defect may be detected by analyzing alteration in activity and/or localization of specific enzymes synthesizing or degradating glycan structures to create terminal GlcNAc-structures. The present description is especially directed to the analysis of the specific glycosylation defect by genetic and/or biochemical and/or cell biological means when the method is verified with the analysis of carbohydrate structures disclosed in the present description, preferably the carbohydrate structures are analyzed by mass spectrometry and/or with known chemical and/or enzymatic methods and/or by substances disclosed in the present description specifically binding and/or modifying the terminal GlcNAcβ-structures disclosed in the present description.

Tumor and cancer specific defective terminal protein linked GlcNAcβ-structures

The present description is especially directed to human cancer specific protein linked GlcNAcβ-structures. The data of the present application shows that protein fractions isolated from certain tumors express many terminal GlcNAc-structures on N-glycan and O-glycan type, especially "specifically protein linked GlcNAcβ-structures", which means that the GlcNAc-structures are present in core structures of N-glycans or O-glycans and not elongated as lactosamine structures. For example the experiment about the protein fraction from the same hypernephroma tumor previously shown to contain glycolipid linked GlcNAcβGal-structures, was now shown to express much of the specifically protein linked GlcNAcβ-structures such as N-glycan with terminal GlcNAcβ2Man-terminals. The present description is thus especially directed to the specified N-glycans and O-glycans and protein linked GlcNAc structures as "specifically protein linked GlcNAc structures". The preferred structures form a specific family of terminal protein linked GlcNAcβ-structures which are human protein linked defective glycosylations present in human cancer. The present description further disclose specific preferred N-glycan structures and O-glycan structures. The preferred terminal beta-GlcNAc O-glycans and N-glycans form a specific family of human cancer specific "protein linked GlcNAcβ-glycan cores" which are result of defective galactosylation of cancer or tumor tissue.

The present application further describes human natural antibodies and more specifically human cancer associated antibodies specifically recognizing preferred human terminal beta-linked GlcNAc structures. The application also describes an enzyme based targeting of the cancer antigens by transferring a modified monosaccharide derivative on cancer cells.

1. Incomplete, undergalactosylated or degraded N-linked glycans.

The N-glycans described here belong to specifically protein linked GlcNAcβ-structures and protein linked Glc-NAcβ-glycan cores disclosed in the present description. The structures include the natural N-glycans and terminal structure containing fragments thereof useful as for immunization or as a binding epitope for targeting substances. All structures contain specific GlcNAcβ2Man-structure.

The inventors have characterized by mass spectrometry several N-glycan structures comprising terminal Glc-NAc residues from tumors such as larynx, stomach, colon and lung tumors. The present description shows that the overexpression of the N-glycans on tumors is common. The novel N-glycan type tumor antigens were also detected specifically by novel glycosyltransferase methods from tissue sections of tumors but not or in lower amounts in corresponding normal or non-malignant tissues.

The present description is directed to N-glycan structure

GlcNAcβ2Manα3(GlcNAcβ2Manα6)Manβ4GlcNAcβ4(Fucα6)GlcNAcβAsn and oligosaccharide substructures thereof carrying non-reducing end protein or peptide linked terminal GlcNAc. Asn indicates asparagine amino acids directly linked to the protein in the natural antigen. The natural protein linked oligosaccharide should contain the reducing end GlcNAcβAsn- structure and at least one of the branches carrying the terminal GlcNAcβ2Man. When the N-glycan structure is used for making antigenic epitopes, the present description is directed to at least one of natural oligosaccharide sequence structures and structures truncated from the reducing end of the N-glycan according to the following

Formula        $[GN\beta2Man]_{r1}\alpha3([GN\beta2Man]_{r2}\alpha6)\{Man[\beta4GN[\beta4(Fuc\alpha6)_{r3}GN]_{r4}]_{r5}\}_{r6}$

wherein

r1, r2, r3, r4, r5, and r6 are either 0 or 1,
with the proviso that at least r1 is 1 or r2 is 1.
GN is GlcNAc, with the proviso that when both r1 and r2 are 1, one GNβMan can be further elongated by one or several other monosaccharide residues such as galactose, and/or one GNβ2Man can be truncated to Man, and/or Manα6-residue and or Manα3 residues can be further substituted by GNβ6 or GNβ4,and/or Manβ4 can be further substituted by GNβ4.

The structures represent truncated forms of known N-linked glycan structures on human N-glycans. Such structures are rare on normal tissues and thus the structures are suitable for immuno diagnostics.
A group of more preferred structures are represented by formula:

$[GN\beta2Man]_{r1}\alpha3([GN\beta2Man]_{r2}\alpha6)\{Man[\beta4GN]_{r5}\}_{r6}$

wherein

r1, r2, r5, and r6 are either 0 or 1,
with the proviso that at least r1 is 1 or r2 is 1.
GN is GlcNAc, with the proviso that when both r1 and r2 are 1, one GNβMan can be further elongated by one or several other monosaccharide residues such as galactose, and/or one GNβ2Man can be truncated to Man, and/or Manα6-residue and/or Manα3 residues can be further substituted by GNβ6 or GNβ4,and/or Manβ4 can be further substituted by GNβ4.

In a more preferred alternative GN is GlcNAc, with the proviso that when both r1 and r2 are 1, one GNβMan can be further elongated by one or several other monosaccharide residues such as galactose, and/or one GNβ2Man can be truncated to Man, and/or Manα6-residue,
and most preferably GN is GlcNAc.
The preferred non-elongated structures include:

GlcNAcβ2Man, GlcNAcβ2Manα3(GlcNAcβ2Manα6)Man,
GlcNAcβ2Manα3(GlcNAcβ2Manα6)Manβ4GlcNAc,
GlcNAcβ2Manα3(GlcNAcβ2Manα6)Manβ4GlcNAcβ4GlcNAc,
GlcNAcβ2Manα3(GlcNAcβ2Manα6)Manβ4GlcNAcβ4(Fucα6)GlcNAc,
GlcNAcβ2Manα3(Manα6)Man, GlcNAcβ2Manα3(Manα6)Manβ4GlcNAc,
GlcNAcβ2Manα3(Manα6)Manβ4GlcNAcβ4GlcNAc,
GlcNAcβ2Manα3(Manα6)Manβ4GlcNAcβ4(Fucα6)GlcNAc,
Manα3(GlcNAcβ2Manα6)Man, Manα3(GlcNAcβ2Manα6)Manβ4GlcNAc,
Manα3(GlcNAcβ2Manα6)Manβ4GlcNAcβ4GlcNAc,
Manα3(GlcNAcβ2Manα6)Manβ4GlcNAcβ4(Fucα6)GlcNAc, GlcNAcβ2Manα3Man,
GlcNAcβ2Manα3Manβ4GlcNAc, GlcNAcβ2Manα3Manβ4GlcNAcβ4GlcNAc,
GlcNAcβ2Manα3Manβ4GlcNAcβ4(Fucα6)GlcNAc, GlcNAcβ2Manα6Man,
GlcNAcβ2Manα6Manβ4GlcNAc, GlcNAcβ2Manα6Manβ4GlcNAcβ4GlcNAc,
GlcNAcβ2Manα6Manβ4GlcNAcβ4(Fucα6)GlcNAc.

More preferred N-glycan oligosaccharide sequences include:

GlcNAcβ2Man, GlcNAcβ2Manα3(GlcNAcβ2Manα6)Man,

GlcNAcβ2Manα3(GlcNAcβ2Manα6)Manβ4GlcNAc, GlcNAcβ2Manα3(Manα6)Man, GlcNAcβ2Manα3(Manα6)Manβ4GlcNAc, Manα3(GlcNAcβ2Manα6)Man, Manα3(GlcNAcβ2Manα6)Manβ4GlcNAc, GlcNAcβ2Manα3Man, GlcNAcβ2Manα3Manβ4GlcNAc, GlcNAcβ2Manα6Man, GlcNAcβ2Manα6Manβ4GlcNAc.

And most preferred N-glycan oligosaccharide sequences include:

GlcNAcβ2Man, GlcNAcβ2Manα3(GlcNAcβ2Manα6)Man, GlcNAcβ2Manα3(GlcNAcβ2Manα6)Man4GlcNAc, GlcNAcβ2Manα3(Manα6)Man, GlcNAcβ2Manα3(Manα6)Manβ4GlcNAc, Manα3(GlcNAcβ2Manα6)Man, Manα3(GlcNAcβ2Manα6)Manβ4GlcNAc.

## 2.Incomplete,undergalactosylated or degraded O-linked glycans.

The O-glycans described here belong to specifically protein linked GlcNAcβ-structures and protein linked Glc-NAcβ-glycan cores disclosed in the present description. The structures include the natural O-glycans and terminal structure containing fragments thereof useful as for immunization or as a binding epitope for targeting substances. All structures contain specific GlcNAcβGalNAc, preferably GlcNAcβGalNAcα-structure.

The inventors have also found out that O-glycans of tumors contain structures carrying terminal β-linked GlcNAc. The O-glycan specificity for tumor is demonstrated in the examples by describing specific natural cancer associated antibody from a person recovered from ovarian cancer and by absence of the antibody in a pool of sera from persons without cancer background.

The present description is specifically directed to human tumor specific O-glycan core structures comprising terminal β-linked GlcNAc residues, preferably with the provision that the O-glycan sequences do not comprise GalNAc-GalNAc sequence. The preferred O-glycan oligosaccharide sequences comprise at least one oligosaccharide sequence according to the formula:

$$[GlcNAc\beta 3]_{s1}[Gal\beta 3]_{s2}(GlcNAc\beta 6)_{s5}GalNAc$$

wherein s1, s2, and s5 are independently 0 or 1, with proviso that at least s1 is 1 or s5 is 1. When there is two GlcNAc structures, one may be substituted with Gal, NeuNAcαGal or other natural oligosaccharide sequences. The Gal-residue may be further substituted with sialic acid or other natural oligosaccharide sequences, in a preferred alternative the Gal-residue is sialylated.

Preferred O-glycan oligosaccharide sequences include: GlcNAcβ3Galβ3(Galβ4GlcNAcβ6)GalNAc, GlcNAcβ3Galβ3(GlcNAcβ6)GalNAc, GlcNAcβ3Galβ3GalNAc, Galβ3(GlcNAcβ6)GalNAc, GlcNAcβ3(GlcNAcβ6)GalNAc, GlcNAcβ6GalNAc, GlcNAcβ3GalNAc, and SAαGalβ3(GlcNAcβ6)GalNAc, wherein SA is sialic acid, preferably Neu5Ac or Neu5Gc or derivative such as O-acetylated derivative thereof which may be α3- or α6-linked to Gal.

More preferably O-glycan oligosaccharide sequences include:

GlcNAcβ3Galβ3GalNAc, Galβ3(GlcNAcβ6)GalNAc, GlcNAcβ6GalNAc and GlcNAcβ3GalNAc and NeuNAcαGalβ3(GlcNAcβ6)GalNAc.

Most preferred O-glycan sequences include Galβ3(GlcNAcβ6)GalNAc, GlcNAcβ3GalNAc , GlcNAcβ6GalNAc and NeuNAcα3Galβ3(GlcNAcβ6)GalNAc.

The O-glycan means that the original antigen is protein/peptide linked from the reducing end to serine or threonine, preferably alpha linked to serine or threonine, or conjugate or analog thereof. The oligosaccharide may also be linked to other carrier aglycon or aglycon spacer structures. Linkage to aglycon or aglycon spacer means that the epitopes are not, at least not directly, linked to carbohydrate, especially not linked to GalNAcα. Aglycon preferably comprises at least one CH2-group like in serine or threonine, giving preferred structures OSα-O-CH$_2$-R, wherein OS is an oligosaccharide according to the present description and R is rest of the aglycon or spacer, preferably comprising additional methylene or acyclic alkyl-structures. In a separate alternative the O-glycan is linked by a beta linkage as decribed above.

The present description is specifically directed to human antibodies recognizing O-glycan oligosaccharide sequence structures Galβ3(GlcNAcβ6)GalNAc, and/or GlcNAcβ6GalNAc but not Galβ3(Galβ4GlcNAcβ6)GalNAc, and/or Galβ4GlcNAcβ6GalNAc and diagnostic uses of these as described by the present description. In a preferred alternative the present description is directed to human antibody recognizing effectively oligosaccharide sequence Galβ3(GlcNAcβ6)GalNAc but not oligosaccharide sequence Galβ3(Galβ4GlcNAcβ6)GalNAc. GlcNAcβ3GalNAc is also preferred O-glycan type target for human antibodies. In preferred alternative the human antibody is natural antibody. In another alternative the antibody is induced by a cancer vaccine. In more preferred alternatives the human antibody is an IgG or IgA or IgM antibody, most preferably a IgG antibody.

As separate alternative the present description is directed to uses of to rare sialylated variants of the O-glycan core structures such as GlcNAcβ3(NeuNAcα6)GalNAc or NeuNAcα3Galβ3(GlcNAcβ6)GalNAc.

3. Poly-N-acetylactosamine type sequences containing terminal GlcNAcβ3 and /or GlcNAcβ6

The polylactosamine sequences described here do no belong to specifically protein linked GlcNAcβ-structures and protein linked GlcNAcβ-glycan cores according to the present description. The structures are useful in combination with the protein specific structures and other specific methods described by the present description.

The present description describes the presence of terminal N-acetylglucosamine (GlcNAc) on poly-Nacetylactosamine type structures on human tumors. The structures were first found in large amounts from a human hyper nephroma tumor in one out of four tumors studied. The glycolipid fraction of the tumor was characterized to contain terminal N-acetylglucosamines by a specific radiolabelled *Escherichia coli* strain and FAB-mass spectrometry of permethylated sample. The glycolipid fraction also contained terminal N-acetyllactosamines, which could be detected by using a specific lectin. Screening of normal kidney glycolipids by the bacterium showed that the terminal GlcNAc was not present in the corresponding normal tissue, as it was not present in several other control tissues.

One alternative of the present description describes detection or isolation of an oligosaccharide sequence or oligosaccharide sequences comprising a terminal N-acetylglucosamine residue from tumor.

Following saccharide sequences are among the tumor specific structures to be isolated or detected: GlcNAcβ3Gal, GlcNAcβ3Galβ4GlcNAc, GlcNAcβ6Gal, GlcNAcβ3 (GlcNAcβ6)Gal, GlcNAcβ3(GlcNAcβ6)Galβ4GlcNAc, GlcNAcβ6Gal or GlcNAcβ6Galβ4GlcNAc, the sequences are part of poly-N-acetyllactosamine chains so that the chains comprise at least one terminal β-linked GlcNAc.

In a more preferred alternative the present description is directed to non-β6- containing linear polylactosamine sequences: GlcNAcβ3Gal, GlcNAcβ3Galβ4GlcNAc, GlcNAcβ3 Galβ4GlcNAc3 Galβ4GlcNAc.

In a separate alternative the present description is directed to β6- containing non-branched polylactosamine sequences: GlcNAcβ6Gal, GlcNAcβ6Galβ4GlcNAc, GlcNAcβ6Galβ4GlcNAc3Galβ4GlcNAc.

A preferred group of poly-N-acetylactosamine type sequences are β3-, β6-branched structures, GlcNAcβ3(GlcNAcβ6)Gal, GlcNAcβ3(GlcNAcβ6)Galβ4GlcNAc, Galβ4GlcNAcβ3(GlcNAcβ6)Galβ4GlcNAc. Then branched structures resemble branched O-glycan structures.

Structures with type one N-acetyllactosamine, GlcNAcβ3Galβ3GlcNAc, or GlcNAcβ6Galβ3GlcNAc are also among the compounds within the scope of the present description.

4. Protein linked N-acetylglucosamine

The protein linked GlcNAc described here belong to specifically protein linked GlcNAcβ-structures according to the present description. The inventors have characterized protein linked GlcNAc residues from tumors such as larynx, stomach, colon and lung tumors. The present description shows that the overexpression of the protein linked GlcNAc expression on tumors is common. The novel N-glycan type tumor antigens were also detected specifically by novel glycosyltransferase methods from tissue sections of tumors but not or in lower amounts in corresponding normal or non-malignant tissues. The analysis of the protein linked GlcNAc indicated presence of several forms of protein linked GlcNAc

In a specific alternative the present description is directed according to the present description to diagnostic uses comprising beta linked N-acetylglucosamine monosaccharide residue, GlcNAcβ. Most of the tumors cells carry the O-glycan like GlcNAc releasable by β-elimination.

The present description is in a preferred alternative directed to the uses according to the present description using O-glycosidic structures

GlcNAcSer and/or GlcNAcThr,

wherein the hydroxyl groups serine and threonine residues are glycosidically linked to the GlcNAc residue. The serine (Ser) and threonine (Thr) amino acid residues are in a preferred alternatives parts of peptides or peptide conjugates or derivatized from amino- and/or carboxylic acid groups.

In another preferred alternative the present description is directed to the uses of GlcNAcX, wherein X is aglycon preferably mimicking serine or threonine amino acid residues described above.

In a preferred alternative GlcNAcβSer and/or GlcNAcβThr, GlcNAcβX is linked to polyvalent carrier according to the present description for uses described by the present description, preferably to a carrier useful for vaccination, and most preferably to a carbohydrate carrier as described by the present description.

In another preferred alternative GlcNAcαSer and/or GlcNAcαThr, GlcNAcαX is linked to polyvalent carrier according to the present description, preferably to a carbohydrate carrier as described by the present description.

The present description is also directed to β-linked N-glycosidic analogs of the O-glycan type structures described above, for example GlcNAcβ-Asn and peptide derivatives and analogs thereof. In biological samples such structures are formed by exoglycosidases or by endo-N-acetylglucosaminyltransferase.

Preferred human specific immune reactions

The present description describes novel natural immune reactions found from persons having cancer or which have been cured from cancer. Possibility of human immune reaction against a novel carbohydrate cannot be guessed from animal data due to species specificity of glycosylation, The present description is especially directed novel human cancer associated immune reactions and human cancer associated antibodies including corresponding humanized antibodies against O-glycan structures GlcNAcβ3GalNAc, GlcNAcβ6(Galβ3)GalNAc, N-glycan structures comprising terminal GlcNAcβ2Man, especially ones binding GlcNAcβ2Manα3(GlcNAcβ2Manα6)Man, and the protein linked GlcNAc-structure, especially when the antibody binds to GlcNAcβ-O-CH$_2$-R, where in the structure is O-glycosidically linked to serine or threonine or an aglycon represented by R as described for O-glycans. The antibody reactions were especially effective against O-glycan structures. Both igM and IgG antibodies against all structures were observed. The present description is targeted both human IgG and IgM antibodies recognizing the preferred structures.

General structures representing oligosaccharide sequences

The oligosaccharide sequences of the present description can be a part of a glycolipid, a part of a glycoprotein, and/or a part of a N-acetyllactosamine chain. The tumor specific oligosaccharide sequences can also be a part of glycolipids, a part of N-linked glycans or O-linked glycans of glycoproteins. The tumor associated oligosaccharide sequences can also be directly linked to O-glycosidic GalNAc. Defects or changes in biosynthetic and/or biodegradative pathways of tumors lead to the synthesis of the oligosaccharide sequences of the present description both on glycolipids and glycoproteins. Terminal N-acetylglucosamine means that the non-reducing end GlcNAc residue in an oligosaccharide chain is not substituted by any other monosaccharide. The term oligosaccharide sequence indicates that the monosaccharide residue/residues in the sequence are part of a larger glycoconjugate, which contains other monosaccharide residues in a chain, which may be branched, or natural substituted modifications of oligosaccharide chains. The oligosaccharide chain is normally conjugated to a lipid anchor or to a protein. In a preferred alternative the the oligosaccharide sequences according to the present description are non-reducing terminal oligosaccharide sequences, which means here that the oligosaccharide sequences are not linked to other monosaccharide or oligosaccharide structures except optionally from the reducing end of the oligosaccharide sequence. The oligosaccharide sequence when present as conjugate is preferably conjugated from the reducing end of the oligosaccharide sequence, though other linkage positions which are tolerated by the antibody/binding substance binding can also be used. In a more specific alternative the oligosaccharide sequence according to the present description means the corresponding oligosaccharide residue which is not linked by natural glycosidic linkages to other monosaccharide or oligosaccharide structures. The oligosaccharide residue is preferably a free oligosaccharide or a conjugate or derivative from the reducing end of the oligosaccharide residue.

Minor species of ganglio- or galactosylglobosides can also represent the tumor specific terminal GlcNAc: terminal Galβ4GlcNAcβ3/β6 structures are linked to the glycolipid cores in some tissues and under low galactosylation conditions described by the present description terminal GlcNAcs can be revealed.

In another alternative of the present description the tumor specific oligosaccharides are detected for the diagnostics of cancer or tumor.

Preferably the tumor specific oligosaccharide sequence is detected by a specific binding substance which can be an aptamer, lectin, peptide, or protein, such as an antibody, a fragment thereof or genetically engineered variants thereof. More preferably the specific binding substance is divalent, oligovalent or polyvalent. Most preferably the binding substance is a lectin or an antibody.

Specific binding combinatorial chemistry libraries can be used to search for the binding molecules. Saccharide binding proteins, antibodies or lectins can be engineered, for example, by phage display methods to produce specific binders for the structures of the present description. Labelled bacteria or cells or other polymeric surfaces containing molecules recognizing the structures can be used for the detection. Oligosaccharide sequences can also be released from cancer or tumor cells by endoglycosidase enzymes. Alternatively oligosaccharides can be released by protease enzymes, such as glycopepides. Chemical methods to release oligosaccharides or derivatives thereof include, e.g., otsonolysis of glycolipids and beta-elimination or hydrazinolysis methods to release oligosaccharides from glycoproteins. Alternatively the glycolipid fraction can be isolated. A substance specifically binding to the tumor specific oligosaccharide sequences can also be used for the analysis of the same sequences on cell surfaces. Said sequences can be detected, e.g., as glycoconjugates or as released and/or isolated oligosaccharide fractions. The possible methods for the analysis of said sequences in various forms also include NMR-spectroscopy, mass spectometry and glycosidase degradation methods. Preferably at least two analysis methods are used, especially when methods of limited specificity are used.

Analysis of multiple cancer specific structures simultaneously from mass spectrometric profiles

The present description is especially directed to the analysis and/or comparision of several analytical signals, preferably mass spectrometry signals produced from a sample comprising total fraction of oligosaccharides released from a cancer or a tumor sample. A single mass spectrum of an oligosaccharide fraction comprise a profile of glycosylation and multiple peaks indicating the potential presence of the oligosaccharide sequences and potential presence of cancer specific oligosaccharide sequences and altered levels thereof in comparison to normal tissue sample. The profiles are determined preferably by MALDI-TOF spectrometry as described in Examples. The total oligosaccharide fraction corresponds preferably total fraction of protein oligosaccharides, preferably comprising at least one cancer or tumor specific oligosaccharide sequence according to the present description. In another preferred alternative the total oligosaccharide fraction comprises at least one cancer or tumor specific O-glycosidic and one N-glycosidic oligosaccharide according to the present description. The present description is further directed to analysis of the multiple mass spectrometric signals when the total oligosaccharide fraction released from a cancer or tumor sample after an enzymatic or a chemical digestion step. The enzymatic digestion is preferably performed by a glycosidase enzyme, preferably selected from the group: galactosidase, sialidase, N-acetylhexosaminidase, N-acetylglucosaminidase, fucosidase or mannosidase.

The tumor specific oligosaccharide sequences can also be used for detection and or quantitation of the human antibodies binding to the tumor specific oligosaccharide sequences, for example, in enzyme-linked immunosorbent assay (ELISA) or affinity chromatography type assay formats. The detection of human antibodies binding to the tumor specific oligosaccharide sequences is preferably aimed for diagnostics of cancer, development of cancer therapies, especially cancer vaccines against the oligosaccharide sequences according to the present description, and search for blood donors which have high amounts of the antibodies or one type of the antibody.

The terminal GlcNAc, or preferably GlcNAc$\beta$3/6Gal$\beta$4GlcNAc-type cancer or tumor glycosylation, may be more common in tumors occurring in patients suffering from immunodeficient conditions, e.g., immunodeficiency causing diseases, such as AIDS, or immunodefiency caused by immunosuppressive medication. Kaposi's sarcoma is a common cancer related to AIDS and immunodeficiency. Immunosuppressive medications are used, for instance, with organ transplantations to prevent rejection during kidney, heart, liver or lung transplantations. Malignancies arising during such therapies are in general benign, but they cause often the loss of the precious organ transplant. Some of the potential natural anticancer antibodies may probably also recognize following epitopes: GalNAc$\beta$3Gal$\beta$4GlcNAc, GalNAc$\alpha$3Gal$\beta$4GlcNAc, and Gal$\alpha$3Gal$\beta$4GlcNAc, which have been shown to be similar. The first structure, $X_2$, is more common in persons who belong to a rare variant of p-blood group, these persons may also have less antibodies recognizing GlcNAc$\beta$3Gal$\beta$4GlcNAc structure. Capability to produce antibodies against tumor or cancer specific antigens may vary according to individual differences in immune system. Persons who have recovered from cancer may have especially high amounts of natural anticancer antibodies.

A possible example from the antibody mediated immune reaction against tumor tissue is a total recovery from hypernephroma after surgery of the majority of the tumor. The oligosaccharide sequences with terminal GlcNAcs are potential targets of such immune response.

Targetting terminal GlcNAc-comprising tumor antigens by glycosyltransferases

The present description is also directed to novel method to transfer a modified monosaccharide derivative on cancer cells or tumor for diagnostics. We disclose a method of generating a covalent bond between a toxic agent, label, drug or immunologically active carbohydrate and the surface of a pathogenic cell of a patient, which surface comprises an acceptor structure recognized by a transferase enzyme, comprising the steps of

-    conjugating said toxic agent, label, drug or immunologically active carbohydrate with a donor molecule of the transferase enzyme, and
-    administering the conjugate obtained and optionally said transferase enzyme to the patient for tumor diagnostics, contacting the conjugate obtained to a tumor sample and detecting said label.

The monosaccharide derivatives to be transferred by glycosyltransferases also comprise a glycosidically linked nucleotide residue. The preferred monosaccharide derivatives are 2-modified such as amide derivatives of UDP-galactosamine

A preferred diagnostic monosaccharide derivative is

UDP-GalN[-S]-D,

wherein

S is an optional spacer group
D is derivatizing group including molecular labels such as for example biotin or a fluorecent molecule including, or a toxic agent, prodrug or prodrug releasing substance as described for other cancer or tumor targetting methods.

The spacer is preferably flexible enough to allow the binding of the modified nucleotide monosaccharide to the transferase.

A preferred monosaccharide derivative is UDP- N-(6-biotinamidohexanoyl)galactosamine. A preferred enzyme to be used is a galactosyltransferase which is engineered to transfer effectively 2-modified monosaccharides. Also natural GalNAc/GlcNAc-transferases with similar specificity from animals for example, may also be used.

The present description is especially directed to method to label tumor tissue by biotin by incubating the tissue with UDP-GalN-spacer-biotin and a modified galactosyltransferase.

The present description is in a separate alternative directed to a diagnostic method in which

1. radiolabelled Gal is transferred from radiolabelled UDP-Gal to human tumor tissue by galactosyltransferase, preferably by β4-galactosyltransferase and
2. the radioactivity incorporated to the tissue is used to determine amount of terminal GlcNAc residues on the tumor.

The methods using galactosyltransferases for labelling are effective for all types of terminal β-GlcNAc structures of the present description.

Transfer of modified galactose residue to cancer for diagnosis of terminal GlcNAcβ-structures

The present description is further directed to enzyme based diagnostic targeting and/or labeling terminal GlcNAc-residues *in vivo* or *ex vivo* by transferring a labeled monosaccharide covalently to the GlcNAc residues of material such as protein or tissue sample or cancer tissue expressing any cancer specific terminal GlcNAc containing structure, preferably any cancer or tumor specific structure according to the present description including the polylactosamine, N-glycan, O-glycan and protein linked terminal GlcNAc-structures. In a preferred alternative polylactosamine, N- glycan, or O-glycan type of structure is targeted. The present description is specifically directed to glycosylation reactions of preferred terminal GlcNAc structures according to the present description. Preferably the monosaccharide is transferred by a β-glycosyltransferase, more preferably by β-glycosyltransferase which is β3- or β4-glycosyltransferase and most preferably by a β4-glycosyltransferase. In a preferred alternative the transferase is able to transfer one or several of the monosaccharide residues selected from the group Glc, GlcNAc, Gal and GalNAc and at least one modified derivative thereof. Preferably the transferase is a Hex(NAc)$_r$β4-transferase, wherein Hex is Glc or Gal, and r is 0 or r is 1. Preferred glycosylatransferases include animal and human glycosyltransferase. Human transferases are especially preferred for *in vivo* uses.

The most preferred form of glycosylatransferase is an animal β4-GlcNAc and/or GalNAc-glycosyltransferase comprising structure allowing transfer of 2-modified Gal or GalN or Glc or GlcN. A preferred transferase has been described by Ramakrishnan and Qasba 2002. This bovine β4-galactosyltransferase enzyme has a mutation at a residue allowing the transfer. The present description is also directed to similar animal enzymes containing the same or similar peptide structure at the catalytic site and having characterized to transfer GalNAc and/or GlcNAc from UDP-GalNAc or UDP-GlcNAc. In a preferred alternative the animal enzyme is a variant of human β4-galactosyltransferase, preferably human β4-galactosyltransferase comprising the same mutation.

A preferred enzyme for transferring the substances is a glycosyltransferase capable of transferring of a monosaccharide unit modified to position 2. A mutated animal form of such enzyme was described by Ramakrishnan and Qasba 2002. The present description is especially directed to soluble human glycosyltransferases and active fragments thereof, especially mutated galactosyltransferases, capable of transferring 2-modified monosaccharide structures.

Preferred 2-modified carbohydrate conjugates to be transferred

The specific diagnostic method is directed to transfer of carbohydrate conjugate to terminal GlcNAc as described by the present description when the carbohydrate to be transferred is modified to position 2. The carbohydrate to be transferred has the structure according to the
Formula C1

HexL-S-T

Wherein Hex is hexose preferably Gal or Glc,
L is linking atom on carbon 2 of the hexose preferably oxygen or nitrogen, or carbon or sulphur atom, more preferably L is oxygen or nitrogen, and most preferably nitrogen.
S is spacer group or atom or nothing,
preferably the spacer length is at least 3 atoms, more preferably at least four and most preferably at least 6 atoms. The spacer may be nothing if the T group includes a flexible aliphatic or equivalent chain such as polyalkylether-structure, preferably poly(ethylene glycol), PEG.

T is the group to be transferred or targeted according to the present description. The transferable group was surprisingly found out to be useful even when larger the acetylgroup naturally present on GalNAc or GlcNAc, even larger than 4 carbon atom substance, even larger than 6- carbon substance or 10- carbon substance, the present description is directed to transfer of substances comprising preferably more than 4 carbon atoms, more preferably more than 6 carbon atoms and most preferably more than 10 carbon atoms. According to present description the transferase accepts is also preferred for use in transfer of modified monosacccharides containing more than about 100 Da extra molecular weight, even more than 500 Da, 1000 Da Or even more than 5000 Da. The present description is directed to use of the full capacity of the preferred glycosyltransferases. The capability of the enzyme to transfer the large group allows the transfer of functional structures such as labels. The capability of transfer even PEG-polymer of about 5000 kDA was even more surprising than the transfer of biotin or similar size labels.

The present description is further directed to the nucleotide sugar conjugates for transferring specific substances T according to the present description according to the Formula C2:

Nu-Hex(L-S-T)

Wherein Hex, L, S and T are as above in Formula C1 and
Nu is a nucleotide activating the carbohydrate conjugate according to the present description. Preferred nucleotides includes UDP, GDP, TDP, and ADP depending on the preference of the glycosyltransferase used. Most preferably UDP is used. Preferred glycosylatransferases includes animal and human glycosyltransferase

Preferred nucleotide sugar conjugate

Most preferred nucleotide sugar conjugates are according to the
Formula C3

UDP-Gal(N-S-T)

and
Formula C4
UDP-Glc(N-S-T), wherein the nucleotide is UDP and linking atom is nitrogen
and S and T are spacer as described above.

Pretargetting methods

Present description is further directed to transfer linking groups by using modified monosaccharides according to the present description. The linking group can be later modified by a corresponding linking group linked with a group to be transferred according to the present description. The present description is especially directed to the transfer of chemoselective and protein/tissue compatible linking groups which are preferably effective in water solutions or aqueous buffers. The chemoselectivity means that linking group reacts to another corresponding linking group with selectivity. The protein and tissue compability means that the linking group or its corresponding linking group does not react, or does not essentially react with amino acid residues or other structures present on the material to be targeted under the conditions of the targeting reaction. The protein and tissue compability depends on the chemical groups present on the material. For example when protein to be targeted does not contain free cysteine side chain, thiol chemistry may be applicable. A preferred pair of chemoselective group is a thiol and a thiol reactive group such as for example maleimide. Another preferred pair of desired linking groups is a aldehyde or ketone to be reacted with an aminooxy group. The aminooxy group reacts selectively and effectively the carbonyl substances in aqueous solution.

The present description is directed to substances according to the formulas C1-C3 when the T group to be targeted is a chemoselective and protein/tissue compatible linking group. The inventors found out that the preferred modified galactosyltransferase according to the present description transfers at least certain non-carbon atom comprising linking groups even when the spacer is short such as 2-4 four atoms. In a preferred alternative the present description is directed to substances comprising a short spacer comprising at least 2 carbon, in preferred alternative at least 3 or 4 atoms, atoms and a linking group, preferably when the spacer has 2 carbon atoms the linking group is N-protected aminooxy group. The aminooxy group is preferably transferred in protected form which is cleaved to reactive aminooxy group under conditions compatible with most proteins. In a specific alternative a protected linking group according to the present description is used.

In another pretargeting method the present description is directed to transfer of biotin as a non-covalent pretargetting group. Additional groups can be targeted to biotin by avidin or strepavidin. The present description is specifically directed to transfer of biotin on to tissue or cell as described by the present description, the examples represent useful methods and reagents and actual labelling.

Methods to modify substrates containing terminal GlcNAc structures in vitro

The present description is aimed for modification of under galactosylated glycoproteins produced by certain cell line, for example by insect cells. The present description is especially directed to modification of terminal GlcNAc containing proteins produced under conditions yielding undergalactosylated (and sialylated) glycoproteins. The lack of terminal glycosylation may be induced by cell culture conditions, such as amount oxygen, presence of monosaccharide substrates and proteins. The present description is further directed to removal of the terminal glycosylation by expressing galactosidase or sialidase (neuraminidase) or fucosidase or N-acetylhexosminidase enzyme, preferably a beta-galactosidase and optionally alpha-neuraminidase to the cell culture medium by the same or different production cell line. The alpha neuraminidase may be of general broad specificity or specific for the sialic acid species produced with alpha3-, or alpha6- and optionally also for alpha8- or alpha 9-linked sialic acid. The glycidase enzyme may be produced as immobilized in the cell wall of yeast or other production cell type.

The present description is directed to modification of terminal GlcNAc on O-glycan or N-glycan and/or protein linked glycans. The substrate of the modification is usually part of regular N-glycosidic or O-glycosidic core structures. The present description is specifically directed to substances according to
Formula P1:

$$\text{Hex(L-S-PEG)-GlcNAc}\beta\text{-Core-peptide}$$

wherein Hex, L and S are as described in Formula C1, Core is a core structure of N-glycan and/or O-glycan, when GlcNAc is part of the core structure the core is the glycan core excluding a terminal GlcNAc residue, PEG is polyethylene glycol, and peptide is protein.

More preferably the present description is directed to substances according to the Formula P2:

$$\text{Gal(N-S-PEG)-GlcNAc}\beta\text{-Core-peptide,}$$

wherein S, PEG-core and peptide are as described above.

In a preferred alternative the present description is preferably directed to substances according to the Formula P3:

$$\text{Glc(N-S-PEG)-GlcNAc}\beta\text{-Core-peptide,}$$

wherein S, PEG-core and peptide are as described above.

The present description is further directed to modified cell or tissue material, when the modification is produced by transfer of modified carbohydrate on the surface of the cell and /or tissue according to the present description. Preferred substances are according to the present description as follows.

$$\text{Formula CT1:} \qquad \text{Hex(L-S-PEG)-GlcNAc}\beta\text{-Core-cell/tiss}$$

wherein Hex, PEG, L and S are as described in Formula D1,
Core is a core structure of N-glycan and/or O-glycan, optionally excluding a terminal GlcNAc residue, Cell/tiss means cell or tissue modifiable.

More preferably the present description is directed to tissues according to the
Formula CT2:

Gal(N-S-PEG)-GlcNAcβ-Cell/tiss,

wherein S, PEG-core and peptide are as described above.

In a preferred alternative the present description is preferably directed to substances according to the Formula CT3:

Glc(N-S-PEG)-GlcNAcβ-Cell/Tiss,

wherein S, PEG-core and peptide are as described above.

The present description is further directed to modified cell or tissue material, when the modification is produced by a transfer of modified carbohydrate on the surface of the cell and /or tissue according to the present description.

The present description especially relates to the in vitro transfer of donor carbohydrate to oligosaccharides related to pathogenic conditions, like cancer specific oligosaccharide sequences, or to those present on pathogens like viruses, bacteria or parasites.

*Detection and diagnostics*

Furthermore the present description is directed to methods for the detection of the pathogenic entities or activities by the present description. The specific transfer of modified monosaccharides to the pathogenic entities allows the detection of the pathogenic entities. For this purpose the modification of the monosaccharide need not to be toxic. The monosaccharide is modified by a label substance like a tag substance including for example an antigen detectable by an antibody, biotin, digotoxigenin, digitoxin or a directly detectable substance with examples of fluorescent substance like rhodamine or flourescein or substance with chemiluminesence activity or phosphorence substance or a specific molecular mass marker detectable by mass spectrometry.

In a preferred alternative the modified monosaccharide is labeled with two label compounds, which are more preferentially a tag substance and a directly detectable substance and most preferentially a tag substance like biotin and a mass spectrometry label. The label substance is preferentially linked through a spacer to the modified monosaccharide. The present description is also directed to the use of said carbohydrate for diagnostics of the pathogenic entities and diseases related to them. The present description is specifically directed to the use of said carbohydrate/carbohydrates for diagnostics of infections, cancer and malignancies. Preferentially the cell surface carbohydrates are labeled by the modified monosaccharide. Modified monosaccharides aimed for detection are useful for detection of certain congenital disorders of glycosylation and under-sialylated LDL. Especially useful are labeled nucleotide sugars.

In a specific alternative the the mofied monosaccharide are labeled with biotin and mass spectrometric labels. The modified monosaccharide are transferred by glycosyltransferring enzyme or enzymes, preferentially by a β4galactosyltransferase, to cell prepararations, the biotinylated fraction is purified and analyzed by mass spectrometry to reveal difference of expression of the glycosyltransferase acceptors. Preferentially glycopeptides produced by proteolysis of the sample are analyzed and recognized by mass spectrometry. In a preferred alternative the method is used to detect difference of O-linked GlcNAc expression levels in cells under different conditions. Preferentially the differences in the same cell or tissue type are detected under different conditions. For the detection of the O-linked N-acetylglucosamine the intracellular proteins should be accessible in the cell preparation. The O-linked GlcNAc is known to be associated with different pathogenic conditions like diabetes.

The target oligosaccharide sequences of the present description can be a part of a glycolipid, a part of a glycoprotein, and/or a part of a N-acetyllactosamine chain. The oligosaccharide sequences on glycoproteins can also be a part of N-linked glycans or O-linked glycans of glycoproteins. Defects or changes in biosynthetic and/or biodegradative pathways of tumors lead to the synthesis of the oligosaccharide sequences of the present description both on glycolipids and glycoproteins. Terminal N-acetylglucosamine means that the non-reducing end GlcNAc residue in an oligosaccharide chain is not substituted by any other monosaccharide. The term oligosaccharide sequence indicates that the monosaccharide residue/residues in the sequence are part of a larger glycoconjugate, which contains other monosaccharide residues in a chain, which may be branched, or natural substituted modifications of oligosaccharide chains. The oligosaccharide chain is normally conjugated to a lipid anchor or to a protein.

Screening of substances binding to the tumor specific terminal GlcNAcs

The present description is specifically directed to the use of the tumor specific terminal-GlcNAc-oligosaccharide sequences for the screening of specific binders with regard to the structures. The screening allows finding of optimal binding substances for the tumor specific oligosaccharide sequences according to the present description. The specific binders may be therapheutic or diagnostic antibodies or other molecules binding to the glycans as described by the present description above.

The screening of the substances binding to the oligosaccharide sequences according to the present description may be performed in an enzyme linked immonoassays (so called ELISA-assays). Direct binding can be measured for example when either the binding substance or the terminal-GlcNAc-glycan structure is linked to a solid phase matrix.

Free oligosaccharides or oligosaccharide conjugates according to the present description can be also used as specific inhibitors in the assays. Fluoresence polararization difference and NMR are examples of liquid phase methods to be used for screening of the substances binding to the oligosaccharide sequences according to the present description.

Use of antibodies for the diagnostics of cancer or tumor and for the targetting of drugs to cancer has been described with other antigens and oligosaccharide structures (US 4,851,511; US 4,904,596; US 5,874,060; US 6,025,481; US 5,795,961; US 4,725, 557; US 5,059,520; US 5,171,667; US 5,173,292; US 6,090,789; US 5,708,163; US 5,902,725 and US 6,203,999). Use of cancer specific oligosaccharides as cancer vaccines has also been demonstrated with other oligosaccharide sequences (US 5,102,663; US 5,660,834; US 5,747,048; US 5,229,289 and US 6,083,929).

The substance according to the present description can be attached to a carrier. Methods for the linking of oligosaccharide sequences to a monovalent or multivalent carrier are known in the art. Preferably the conjugation is performed by linking the cancer specific oligosaccharide sequences or analogs or derivatives thereof from the reducing end to a carrier molecule. When using a carrier molecule, a number of molecules of a substance according to the present description can be attached to one carrier increasing the stimulation of immune response and the efficiency of the antibody binding. To achieve an optimal antibody production, conjugates larger than 10 kDa carrying typically more than 10 oligosaccharide sequences are preferably used.

The oligosaccharide sequences according to the present description can be synthesized, for example, enzymatically by glycosyltransferases, or by transglycosylation catalyzed by a glycosidase enzyme or a transglycosidase enzyme, for review see Ernst et al., 2000. Specificities of the enzymes and their use of co-factors such as nucleotide sugar donors, can be engineered. Specific modified enzymes can be used to obtain more effective synthesis, for example, glycosynthase is modified to achieve transglycosylation but not glycosidase reactions. Organic synthesis of the saccharides and conjugates of the present description or compounds similar to these are known (Ernst et al., 2000). Carbohydrate materials can be isolated from natural sources and be modified chemically or enzymatically into compounds according to the present description. Natural oligosaccharides can be isolated from milks of various ruminants and other animals. Transgenic organisms, such as cows or microbes, expressing glycosylating enzymes can be used for the production of saccharides.

It is possible to incorporate an oligosaccharide sequence according to the present description, optionally with a carrier, in a pharmaceutical composition, which is suitable for the treatment of cancer or tumor in a patient. Examples of conditions treatable according to the present description are cancers in which the tumor expresses one or more of the tumor specific oligosaccharides described in the present description. The treatable cancer cases can be discovered by detecting the presence of the tumor specific oligosaccharide sequences in a biological sample taken from a patient. Said sample can be a biopsy or a blood sample.

The term "treatment" used herein relates to both treatment in order to cure or alleviate a disease or a condition, and to treatment in order to prevent the development of a disease or a condition. The treatment may be either performed in a acute or in a chronic way.

The term "patient", as used herein, relates to any mammal in need of treatment.

When a tumor specific oligosaccharide or compound specifically recognizing tumor specific oligosaccharides of the present description is used for diagnosis or typing, it may be included, e.g., in a probe or a test stick, optionally in a test kit. When this probe or test stick is brought into contact with a sample containing antibodies from a cancer patient or cancer cells or tissue of a patient, components of a cancer positive sample will bind the probe or test stick and can be thus removed from the sample and further analyzed.

In the present description the term "tumor" means solid multicellular tumor tissues. Furthermore the term "tumor" means herein premalignant tissue, which is developing to a solid tumor and has tumor specific characteristics. The term "tumor" is not referring herein to a single cell cancer such as a leukaemia or to cultured cancer cells or a cluster of such cells. The present description is preferably directed to primary human cancer samples. It is well known that glycosylations in cultivated cancer cells vary and are not in general relevant with regerd to cancer. It is also known that transfections, cell culture media and dividing solid tumor to single cells may have daramatic effects for glycosylations. The term cancer includes tumors.

Glycolipid and carbohydrate nomenclature is according to recommendations by the IUPAC-IUB Commission on Biochemical Nomenclature (Carbohydr. Res. 1998, 322:167; Carbohydr. Res. 1997, 297:1; Eur. J. Biochem. 1998, 257:29).

It is assumed that Gal, Glc, GlcNAc, and NeuNAc are of the D-configuration, Fuc of the L-configuration, and that all monosaccharide units are in the pyranose form. Glucosamine is referred as GlcN and galactosamine as GalN. Glycosidic linkages are shown partly in shorter and partly in longer nomenclature, the linkages $\alpha 3$ and $\alpha 6$ of the NeuNAc-residues mean the same as $\alpha 2$-3 and $\alpha 2$-6, respectively, and $\beta 1$-3, $\beta 1$-4, and $\beta 1$-6 can be shortened as $\beta 3$, $\beta 4$, and $\beta 6$, respectively. Lactosamine or N-acetyllactosamine or Gal$\beta 3/4$GlcNAc means either type one structure residue Galβ3GlcNAc or type two structure residue Galβ1-4GlcNAc, and sialic acid is N-acetylneuraminic acid or NeuNAc, Lac refers to lactose and Cer is ceramide.

The present invention is further illustrated in examples, which in no way are intended to limit the scope of the invention. The reference examples, not falling within the scope of the claims, but useful for understanding the invention are marked up as "reference".

EXAMPLES

Example 1 (reference).

*Culturing and labelling of bacteria*

The recombinant G-fimbriated *Escherichia coli* strain IHE11088 (pRR-5), expressing the GlcNAc-recognizing GafD adhesin (Rhen, M. et al., 1986), was cultured in Luria broth containing tetracyclin (25 μg/ml) and 10 μl [$^{35}$S]-methionine (400 mCi; Amersham Pharmacia Biotech, Little Chalfont, UK) at 37°C over night. The bacteria were harvested by centrifugation, washed two times with phosphate-buffered saline, pH 7.2 (PBS), and resuspended in PBS to 1 x 10$^9$ CFU/ml. The specific activities were approximately 100 CFU/cpm.

*Labelling of Erythrina cristagalli lectin*

The Galβ4GlcNAcβ-binding lectin from *Erythrina cristagalli* (Teneberg et al., 1994) was purchased from Vector Laboratories Inc., Burlingame, CA. Batches of 100 μg protein were labelled with $^{125}$I, using Na$^{125}$I (100 mCi/ml; Amersham Pharmacia Biotech), according to the IODO-GEN protocol of the manufacturer (Pierce, Rockford, IL). Approximately 5 x 10$^3$ cpm/μg protein was obtained.

*Glycosphingolipid binding assays*

Binding of radiolabeled bacteria and lectin to glycosphingolipids separated on thin-layer chromatograms was done as reported previously (Teneberg et al., 1994, Hansson et al., 1985). Thin-layer chromatography of glycosphingolipids was performed on aluminium-backed silica gel 60 HPTLC plates (Merck, Darmstadt, Germany), using chloroform/methanol/water 60:35:8 (by volume) as solvent system. Dried chromatograms were dipped for 1 min in diethylether/n-hexane 1:5 (by volume) containing 0.5% (w/v) polyisobutylmethacrylate (Aldrich Chem. Comp. Inc., Milwaukee, WI). After drying, the chromatograms were soaked in PBS containing 2% bovine serum albumin (BSA) (w/v), 0.1% NaN$_3$ (w/v) for 2 hr at room temperature. The chromatograms were subsequently covered with radio labelled bacteria diluted in PBS (2-5 x 10$^6$ cpm/ml) or radiolabelled lectin in BSA (2 x 10$^3$ cpm/ml). Incubation was done for 2 hr at room temperature, followed by repeated washings with PBS. The chromatograms were thereafter exposed to XAR-5 X-ray films (Eastman Kodak, Rochester, NY) for 12 hr.

Example 2 (reference).

*Demonstration of tumor specificity of terminal GlcNAc structure.*

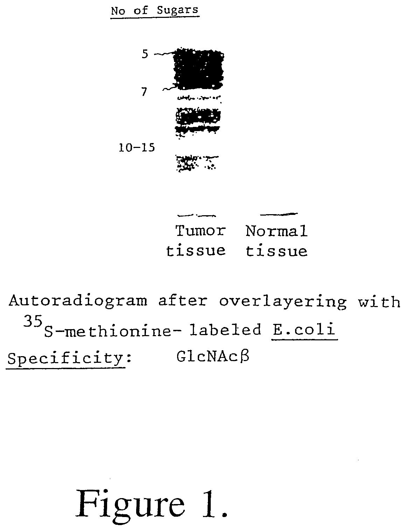

Thin-layer overlay assays were performed with radiolabelled G-fimbriated *Escherichia coli* to screen various tumors and normal tissues. The *E.coli* strain IHE11088 (pRR-5) specifically recognizes terminal GlcNAcβ-structures (Rhen, M. et al. 1986). Binding active glycolipids were found in non-acid fraction from one of four hypernephroma tumors studied (Fig 1). No binding was observed towards corresponding fraction of non-acid glycosphingolipids from human normal kidney or to other control tissues studied.

Example 3 (reference).

*Characterizations of terminal GlcNAcβ-suctures from human hypernephroma.*

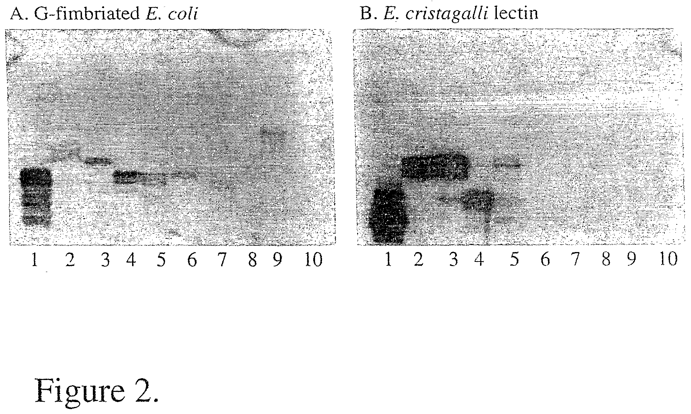

Non-acid glycosphingolipids from human hypernephroma tumor were fractionated and analysed by binding with the GlcNAcβ-specific G-fimbriated *E.coli* (Fig.1A) and lectin from *Erythrina cristagalli* which recognizes terminal Galβ4GlcNAcβ-structures (Fig. 2B) by thin-layer overlay assay. The two binding reagents show partially overlapping glycospingolipid binding species. The data indicates that the terminal GlcNAc-species are mostly present on N-acetyllactosamine type non-acid glycosphingolipids. The terminal GlcNAc-species which do not have an overlap with lectin binding activity have most probably terminal structures where N-acetyllactosamine is derivatized by GlcNAc such as GlcNAcβ3Galβ3/4GlcNAcβ-; diffuse bands probably also indicates the presence of an isomeric form GlcNAcβ6Galβ3/4GlcNAcβ-. The sample also appears to contain minor species where the terminal GlcNAc and N-acetyllactosamine are present in the same glycolipid. This indicates the presence of branched structures such as Galβ3/4GlcNAcβ3(GlcNAcβ6)Galβ3/4GlcNAcβ- and GlcNAcβ3(Galβ3/4GlcNAcβ6)Galβ3/4GlcNAcβ-; the size distribution of the glycosphingolipids probably also indicates species with two or even more terminal GlcNAcs. The binding of the *Erythrina cristagalli* lectin indicates that most of the lactosamine probably has the type two N-acetyllactosamine structure Galβ4GlcNAc.

The glycolipids were partially analyzed by FAB mass spectrometry and by EI masspectrometry after permethylation, which showed presence of terminal HexNAc and that the smallest species with terminal GlcNAc is a pentasaccharide glycosphingolipid probably of lacto or neolacto series. Also 7-meric and larger structures up to 15-mer were observed (Fig. 1). The binding of the lectins indicates that most of the lactosamine probably has the type two N-acetyllactosamine structure.

Example 4.

*Materials and methods for protein linked structures and labeling by galactosyltransferase*

*Isolation of glycans from formalin-fixed or formalin-fixed and paraffin-embedded tissue samples.* Prior to glycan isolation from formalin-fixed samples, proteins were enriched by chloroform-methanol extraction essentially as described in (Manzi *et al.,* 2000). Quantitative extraction of glycoproteins was confirmed by radioactively labelled glycoprotein standards (not shown). Prior to glycan isolation from formalin-fixed and paraffin-embedded samples, the samples were deparaffinised. Glycans were detached from sample glycoproteins by non-reductive β-elimination and purified by chromatographic methods.

*MALDI-TOF MS.* MALDI-TOF mass spectrometry was performed with a Voyager-DE STR BioSpectrometry Workstation, essentially as in (Saarinen et al., 1999; Papac et al., 1996; Harvey, 1993).

*Exoglycosidase digestions.* All exoglycosidase reactions were performed essentially as described in (Nyman et al., 1998; Saarinen et al., 1999) and analysed by MALDI-TOF MS. The enzymes and their specific control reactions with characterised oligosaccharides were: β-N-acetylglucosaminidase (*Streptococcus pneumoniae,* recombinant, *E. coli;* Calbiochem, USA) digested GlcNAcβ1-6Gal-R but not GalNAcβ1-4GlcNAcβ1-3/6Gal-R; β1,4-galactosidase (*Streptococcus pneumoniae,* recombinant, *E. coli;* Calbiochem, USA) digested Galβ1-4GlcNAc-R but not Galβ1-3GlcNAc-R; α-mannosidase (Jack bean; Glyko, UK) transformed a mixture of high-mannose N-glycans to the $Man_1GlcNAc_2$ N-glycan core trisaccharide. Control digestions were performed in parallel and analysed similarly to the analytical exoglycosidase reactions.

*Synthesis of UDP-GalN-biotin.* UDP-galactosamine (UDP-GalN) is formed from UDP-Glc and galactosamine-1-phosphate by the action of galactose-1-phosphate uridyltransferase (E.C. 2.7.7.12; Sigma, USA). A typical synthesis protocol is described below. The reaction mixture contains 10 mM galactosamine-1-phosphate, 20 mM UDP-Glc, 5 U/ml of galactose-1-phosphate uridyltransferase, 100 mM Na-HEPES pH 8.0, 5 mM $MgCl_2$, and 5 mM β-mercaptoethanol. The reaction vessel is incubated at room temperature under nitrogen atmosphere for 3 days, after which nucleotide sugars are isolated from the reaction mixture with a graphitised carbon column essentially as in (Mäki *et al.,* 2002). The nucleotide sugar mixture, containing UDP-Glc and UDP-GalN, is incubated with a molar excess of sulfosuccinimidyl-6-(biotinamido)hexanoate (sulfo-NHS-LC-biotin; Pierce, USA) in 50 mM $NH_4HCO_3$ at room temperature for 2.5 hours. The product, UDP-GalN-biotin, uridine 5'-diphospho-N-(6-biotinamidohexanoyl)galactosamine, is purified by gel filtration and reversed phase HPLC.

*Labeling of terminal GlcNAc residues in oligosaccharides and tissue sections with UDP-GalN-biotin.* N-(6-biotinamidohexanoyl)galactosamine can be transferred from UDP-GalN-biotin to a terminal GlcNAc containing acceptor with a recombinant β1,4-galactosyltransferase similar to the enzyme described in (Ramakrishnan and Qasba, 2002). In a typical procedure, an oligosaccharide acceptor such as GlcNAcβ1-3Galβ1-4GlcNAcβ1-3Galβ1-4Glc, or deparaffinised formalin-fixed paraffin-embedded tissue sections, are incubated at +37 °C with a reaction mixture containing 10 mM UDP-GalN-biotin, 160 U/l enzyme, 100 mM Tris-HCl, and 20 mM $MnCl_2$. After washing, the biotin groups are visualised by standard methods in the art, using streptavidin or avidin coupled reagents, for example streptavidin-FITC.

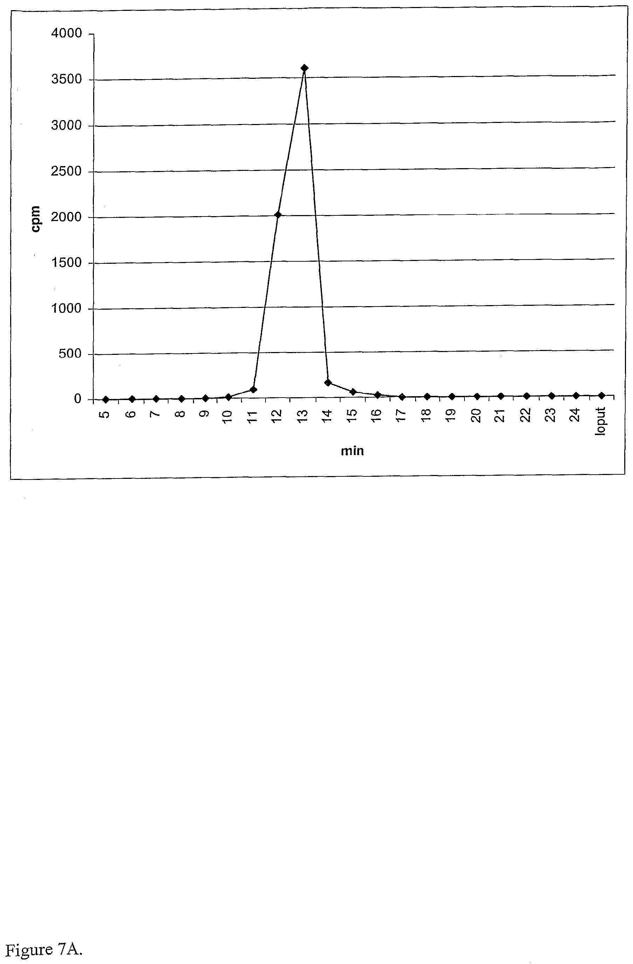

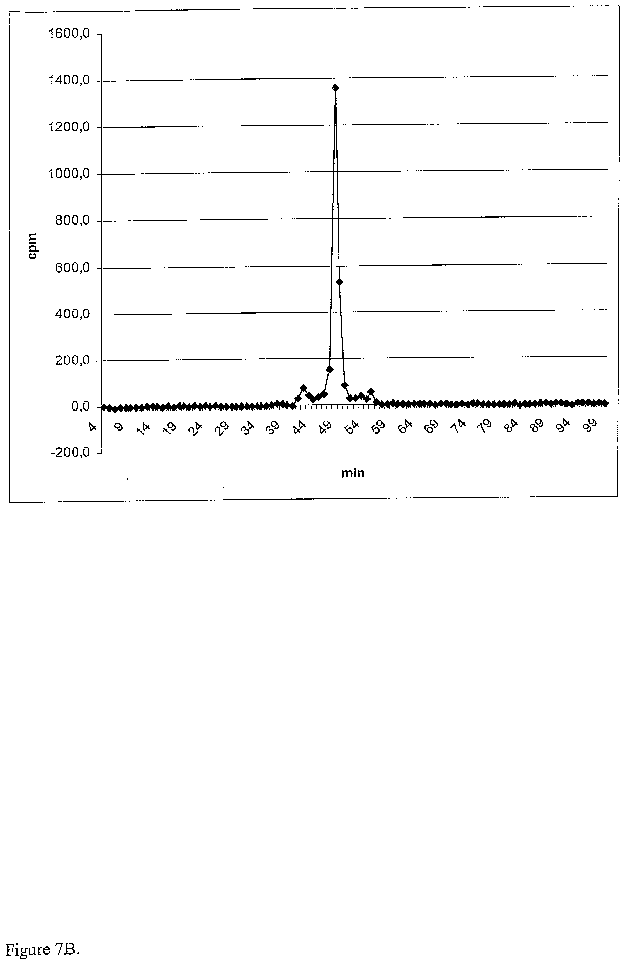

*Labeling of terminal GlcNAc residues in tissue sections with UDP-[14C]Gal.* Formalin-fixed and paraffin-embedded tissue sections were deparaffinised and incubated at +37 °C with a reaction mixture containing UDP-[14C]Gal, 200 U/l bovine milk β1,4-galactosyltransferase (Calbiochem, USA), 50 mM Na-MOPS pH 7.4, and 20 mM $MnCl_2$. After washing, the labelled sections were subjected to autoradiography. N-glycans were detached from the tissue sections with *Chryseobacterium meningosepticum* N-glycosidase F (Calbiochem, USA) essentially as in (Nyman *et al.,* 1998). Chromatography was performed as described in figure legends to Fig. 7A and 7B.

Example 5 (reference)

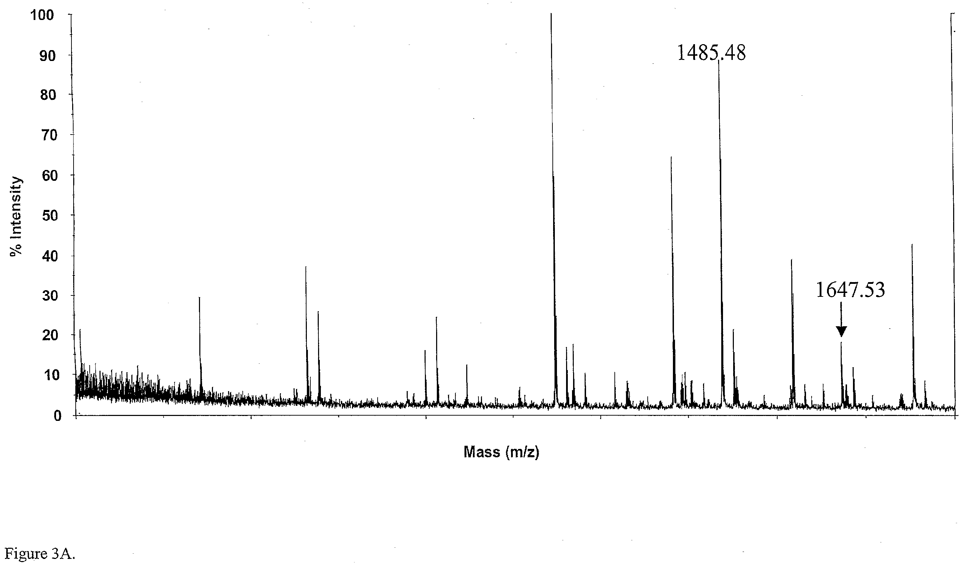

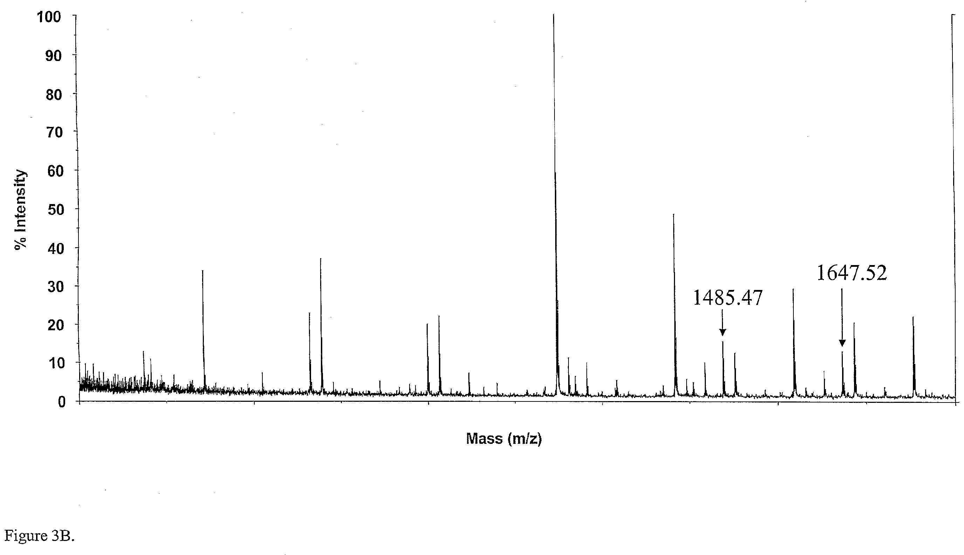

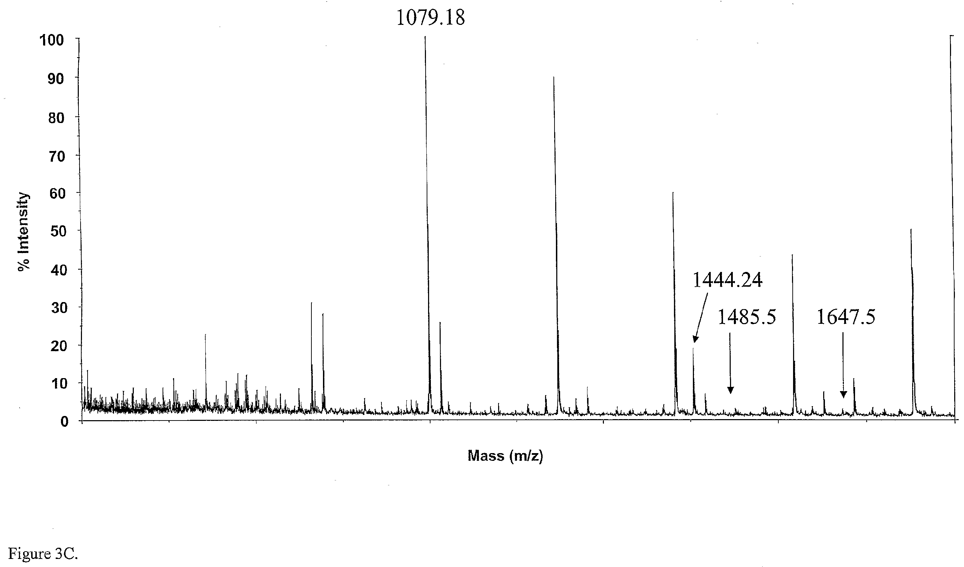

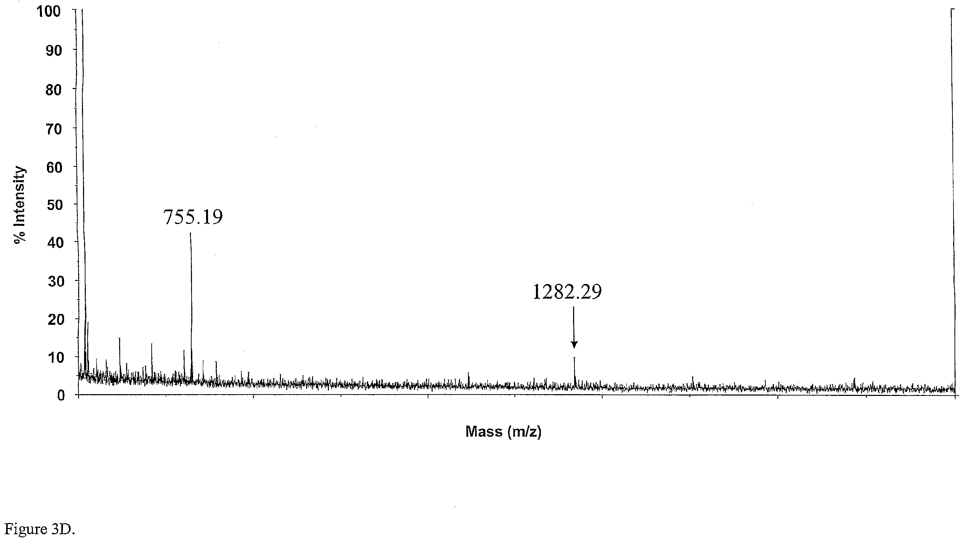

*Cancer-associated terminal GlcNAc containing N-glycans from lung adenocarcinoma samples.* Formalin-fixed samples, from tumor and surrounding healthy tissue, were obtained from a patient with lung adenocarcinoma. There was a significant difference between the neutral glycans isolated from the tumor sample (Fig. 3A) and the healthy tissue sample (Fig. 3B), namely a peak at m/z 1485.48, corresponding to the ion $[Hex_3HexNAc_4Fuc_1+Na]^+$ (calc. m/z 1485.53). The relative intensity of this glycan peak was elevated over 6.1 times in the tumor sample, as compared to healthy tissue. Furthermore, a peak at m/z 1647.53, corresponding to the ion $[Hex_4HexNAc_4Fuc_1+Na]^+$ (calc. m/z 1647.59), had a higher signal intensity in the tumor sample. Upon β-N-acetylglucosaminidase digestion (Fig. 3C), the two peaks were completely transformed into peaks at m/z 1079.18, corresponding to the ion $[Hex_3HexNAc_2Fuc_1+Na]^+$ (calc. m/z 1079.38), and 1444.24, corresponding to the ion $[Hex_4HexNAc_3Fuc_1+Na]^+$ (calc. m/z 1444.51), respectively, indicating the presence of terminal β-GlcNAc residues. Jack bean α-mannosidase digestion (Fig. 3D) further transformed these peaks into peaks at m/z 755.19, corresponding to the ion $[Hex_1HexNAc_2Fuc_1+Na]^+$ (calc. m/z 755.27), and 1282.29, corresponding to the ion $[Hex_3HexNAc_3Fuc_1+Na]^+$ (calc. m/z 1282.45), respectively. However, α-mannosidase digestion before the β-N-acetylglucosaminidase digestion did not affect the two peaks, indicating that the α-Man residues were subterminal to the β-GlcNAc residues. In addition, β1,4-galactosidase digestion of the original neutral glycan sample completely transformed the peak at m/z 1647.5 into the peak at m/z 1485.5, indicating the presence of a terminal Galβ1-4GlcNAc unit. Taken together, the results suggest that the lung adenocarcinoma tumor sample contained highly elevated amounts of the complex N-linked glycan core structure GlcNAcβ-Manα1-6(GlcNAcβ-Manα1-3)Manβ1-4GlcNAcβ1-4(Fucα1-6)GlcNAc, and slightly elevated amounts of the mono-β1,4-galactosylated (to GlcNAc) derivative of the former structure, as compared to the surrounding healthy tissue.

Example 6 (reference).

*Occurrence of the terminal GlcNAc containing N-glycans in carcinoma samples.* The occurrence of the abovementioned structures in various tumor and healthy control samples was studied by isolating and analysing the neutral glycan fractions by MALDI-TOF MS and exoglycosidase digestions. The analysed tumor-control pairs were: 7 lung cancer sample pairs, and one pair each of colon, stomach, and larynx cancer samples. It turned out that in every case the relative abundance of the two terminal GlcNAc containing N-glycan at m/z 1485.5, was elevated in the cancerous sample. However, there were significant individual differences in the expression levels of this glycan epitope both in the healthy state and in cancer. Table 1 summarizes the differential expression of the m/z 1485.5 N-glycan in relation to the bulk of the isolated neutral glycan fraction.

Example 7.

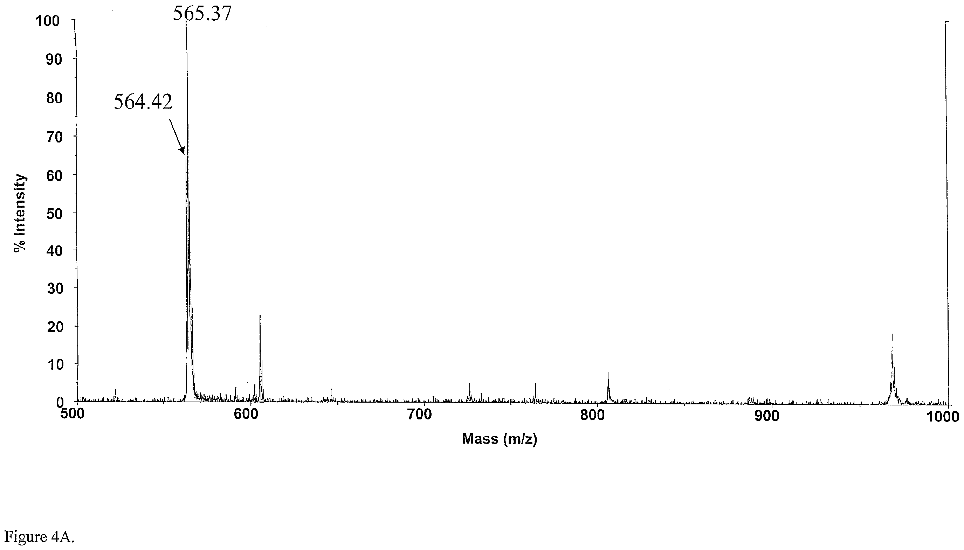

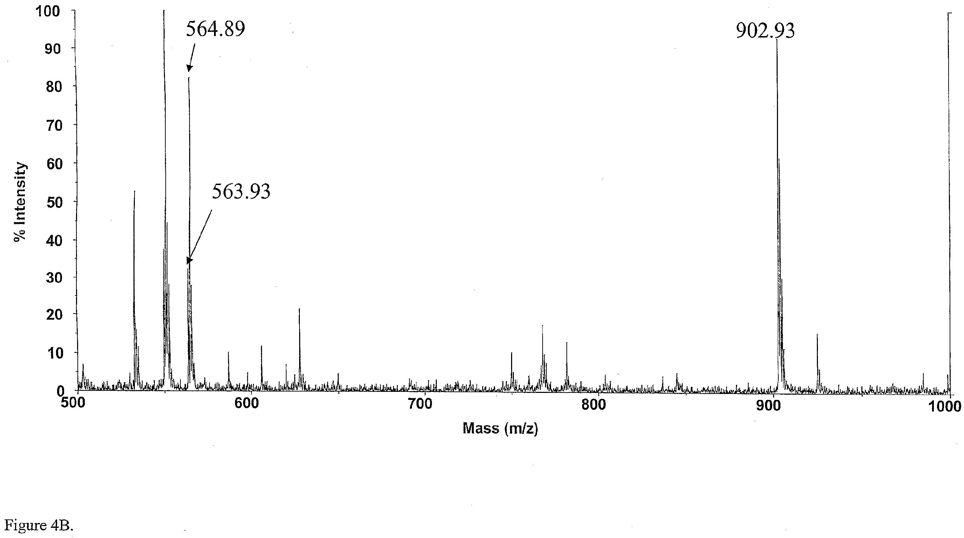

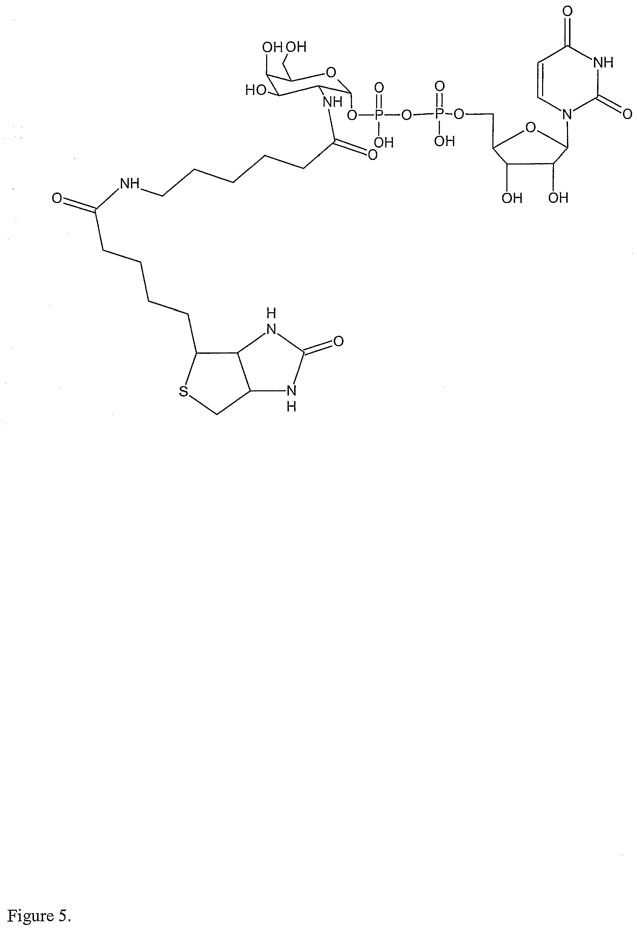

*Synthesis of UDP-GalN-biotin.* UDP-galactosamine was synthesized as described under Materials and Methods. The product was characterized by MALDI-TOF MS (obs. m/z 564.42 for $[C_{15}H_{25}N_3O_{16}P_2-H]^-$, calc. m/z 564.31); Fig. 4A) and the expected peak appeared in the mass spectrum one mass unit smaller than the peak of UDP-Glc (obs. m/z 565.37 for $[C_{15}H_{24}N_2O_{17}P_2-H]^-$, calc. m/z 565.29); Fig. 4A). The crude nucleotide sugar preparate was reacted with a biotinylation reagent, namely succinimidyl-6-(biotinamido)hexanoate. After the reaction, the expected product could be seen in the MALDI-TOF mass spectrum of the reaction mixture (obs. m/z 902.93 for $[C_{31}H_{50}N_6O_{19}P_2S-H]^-$, calc. m/z 903.76; Fig. 4B). UDP-Glc did not react at all with the biotinylation reagent. The synthesized UDP-GalN-biotin, uridine 5'-diphospho-N-(6-biotinamidohexanoyl)galactosamine, reacted with GlcNAcβ1-3Galβ1-4GlcNAcβ1-3Galβ1-4Glc and a recombinant β1,4-galactosyltransferase similar to the enzyme described in (Ramakrishnan and Qasba, 2002). The product, [N-(6-biotinamidohexanoyl)galactosamine]β1-4GlcNAcβ1-3Galβ1-4GlcNAcβ1-3Galβ1-4Glc, was characterized by MALDI-TOF MS (obs. m/z for $[M+Na]^+$ 1433.38, calc. m/z 1433.55). Taken together, the results indicate that the synthesized product has the expected structure (Fig. 5). The product was chromatographically purified to homogeneity.

Example 8.

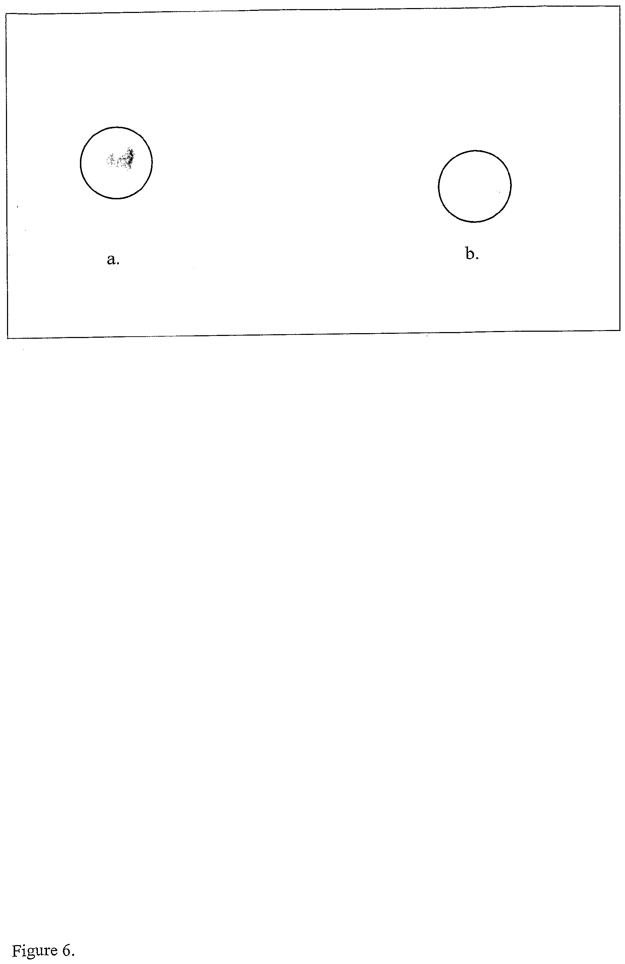

*Labeling of terminal GlcNAc residues in tissue sections with UDP-[14C]Gal and UDP-GalN-biotin.* Deparaffinised formalin-fixed and paraffin-embedded tissue sections were radioactively labeled with UDP-[14C]Gal and bovine milk β1,4-galactosyltransferase, as described under Materials and Methods. Autoradiography revealed a clear difference between the tumor and the healthy tissue samples (Fig. 6), indicating that there are highly elevated amounts of terminal GlcNAc residues in the lung adenocarcinoma sample. Similar results were also obtained by using the UDP-GalN-biotin reagent, as described under Materials and Methods, streptavin-FITC, and fluorescence microscopy. Importantly, cancer cells could be labeled with this biotinylation reagent.

Example 9.

*Isolation of [14C] Gal-labeled oligosaccharides from lung adenocarcinoma sample.* After labeling of lung adenocarcinoma sample and surrounding healthy tissue sections with [14C]Gal as described above, the labeled oligosaccharides were isolated by N-glycosidase F digestion and nonreductive β-elimination. In the gel filtration chromatogram of the N-glycosidase F liberated glycans from lung adenocarcinoma (Fig. 7A), only one peak was visible and it coeluted with the N-glycan standards Galβ1-4GlcNAcβ1-2Manα1-6(Galβ1-4GlcNAcβ1-2Manα1-3)Manβ1-4GlcNAcβ1-4(Fucα1-6)GlcNAc and Galβ1-4[GlcNAcβ1-2Manα1-6(GlcNAcβ1-2Manα1-3)Manβ1-4GlcNAcβ1-4(Fucα1-6)GlcNAc]. The peak was pooled and subjected to HPLC with a porous graphitized carbon column (Fig. 7B), where it was divided into one major and two minor peaks. The major peak, containing nearly all of the total radioactivity, coeluted with the N-glycan standard Galβ1-4[GlcNAcβ1-2Manα1-6(GlcNAcβ1-2Manα1-3)Manβ1-4GlcNAcβ1-4(Fucα1-6)GlcNAc].

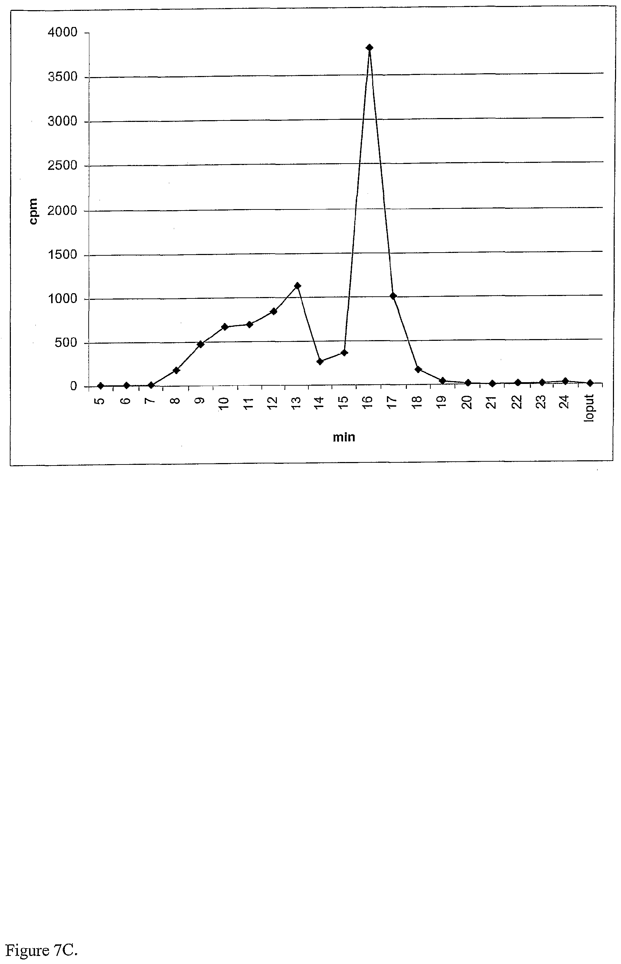

In the gel filtration HPLC chromatogram of the material liberated by nonreductive β-elimination from lung adenocarcinoma (Fig. 7C), a broad peak, containing 45 % of the total radioactivity, was found to elute between the void volume (at 8 ml) and the elution position of the N-glycan standard Galβ1-4[GlcNAcβ1-2Manα1-6(GlcNAcβ1-2Manα1-3)Manβ1-4GlcNAcβ1-4(Fucα1-6)GlcNAc]. The broad peak was pooled and passed through columns of strong cation exchange material and $C_{18}$ silica, which would retain all glycopeptidic material, but allow for quantitative elution of free oligosaccharides. Nearly 80 % of the radioactivity in the pooled fractions was retained in the columns, indicating that the broad peak indeed corresponded to alkali-liberated glycopeptides, from which the [14C]Gal labeled glycan moieties had not been detached. Major part of the remaining radioactivity was found to correspond to the N-glycan structure described above, but the presence of other labelled oligosaccharides could not be excluded. The major peak in the gel filtration HPLC chromatogram, containing 55 % of the total radioactivity, coeluted with an N-acetyllactosamine (LacNAc) standard.

Example 10.

*Protein linked GlcNAc from cancer samples*

Furthermore, in graphitized carbon column HPLC of the pooled fractions at 15 - 18 min, the major peak coeluted with LacNAc. This suggests that the sample contains base-labile GlcNAc monosaccharide-protein conjugates, most likely GlcNAcβ-*O*-Ser/Thr units. Importantly, the amount of [14C]-labeled LacNAc was significantly (1.99 times) higher in the lung adenocarcinoma sample as compared to the surrounding healthy tissue sample.

Taken together, these results indicate that about half of the total radioactivity that can be liberated from UDP-[14C]Gal labeled lung adenocarcinoma sample tissue sections, represents the [14C]Gal labeled forms of the cancer-associated N-glycan GlcNAcβ-Manα1-6(GlcNAcβ-Manα1-3)Manβ1-4GlcNAcβ1-4(Fucα1-6)GlcNAc. Furthermore, it is evident that also in the UDP-GalN-biotin reaction, the label is transferred into the oligosaccharide structures characterized above.

Example 11 (reference).

*Isolation of anti-GlcNAc antibodies from human serum.* Human serum from a person who had recovered from mucinous ovarian adenocarcinoma, was passed through Sepharose columns that contained either GlcNAcβ1-6(Galβ1-3)GalNAcα or Galβ1-4GlcNAcβ1-6(Galβ1-3)GalNAcα epitopes covalently coupled to the gel. After washing, the columns were firstly eluted with a buffer containing 0.5 M GlcNAc (specific elution), and secondly with acidic buffer (unspecific elution). As a control, an IgG preparation from pooled human sera from healthy donors, i.e. persons who did not have a history of malignant diseases, was also subjected to the abovementioned chromatographical procedure. Reducing SDS-PAGE analysis was done to the collected fractions (Fig. 8). From the results it can be seen that two bands corresponding to proteins that had similar relative molecular weights to the heavy and light chains of an IgG standard, were eluted in the specific 0.5 M GlcNAc elution of GlcNAcβ1-6(Galβ1-3)GalNAcα Sepharose, but only in the serum sample of the person who had recovered from cancer. In contrast, no such specific elution could be detected in Galβ1-4GlcNAcβ1-6(Galβ1-3)GalNAcα Sepharose chromatography of the two samples. According to their relative molecular weight, the specifically eluted proteins are likely to represent the heavy and light chain subunits of IgG or IgA, but not IgM human antibodies. The present results indicate the presence of elevated levels of terminal GlcNAc specific antibodies in serum of the person who had recovered from cancer, more specifically IgG and/or IgA antibodies that recognize the GlcNAcβ1-6(Galβ1-3)GalNAcα glycan epitope.

Example 12 (reference). Determination and statistical evaluation of decreased neutral N- and O-glycan galactosylation in human carcinomas.

*Galactosylation degree calculation.*

For galactosylation degree determinations, the non-sialylated glycan fractions from non-small cell lung carcinoma and from control samples from respective healthy lung tissue, were isolated and analyzed by MALDI-TOF mass spectrometry as described above. The galactosylation degrees were calculated as follows. The relative intensities (I) of three peaks in the mass spectra were measured, namely those at m/z 1485.5 (corresponding to the ion $[\text{Hex}_3\text{HexNAc}_4\text{dHex}_1\text{Na}]^+$, with two non-reducing terminal N-acetylglucosamine residues), m/z 1647.6 (corresponding to the ion $[\text{Hex}_4\text{HexNAc}_4\text{dHex}_1\text{Na}]^+$, with one non-reducing terminal N-acetyllactosamine group and one non-reducing terminal N-acetylglucosamine residue), and m/z 1809.6 (corresponding to the ion $[\text{Hex}_5\text{HexNAc}_4\text{dHex}_1\text{Na}]^+$, with two non-reducing terminal N-acetyllactosamine groups). The non-reducing terminal residues of these glycan components were previously determined as either N-acetylglucosamine according to sensitivity to digestion with glucosaminidase from Jack beans, or N-acetyllactosamine according to sensitivity to β-galactosidase digestion. The degree of galactosylation (DG) was calculated as the relation of galactosylated antennae N-acetylglucosamine residues to the total antennae N-acetylglucosamine residues, according to the formula:

$$DG = (\, I_{1647.6} + 2 \times I_{1809.6} \,) : (\, 2 \times I_{1485.5} + 2 \times I_{1647.6} + 2 \times I_{1809.6} \,) \times 100\ \%$$

*Statistical calculations.*

Statistical analyses were performed with the SAS Software (SAS System, version 8.2, SAS Institute Inc., Cary, NC, USA), using SAS/STAT and SAS/BASE modules. All tests were performed as two-sided. The distributions of the experimental data were evaluated as 1) normal and symmetric, 2) only symmetric, or 3) non-symmetric and not normal, and the statistical test used was accordingly chosen as 1) Student's t Test, 2) Wilcoxon Signed Rank Test, or 3) Sign Test. A p value of less than 0.05 was considered statistically significant.

*Galactosylation degree of neutral monofucosylated biantennary N-glycans is significantly reduced in non-small cell lung adenocarcinoma.*

All tumor samples from a randomly picked group of 7 patients with non-small cell lung adenocarcinoma showed a decrease in the galactosylation degrees of neutral monofucosylated biantennary N-glycans, when compared to the respective control samples from the same patients. This difference is statistically significant (p = 0.0156 in Wilcoxon Signed Rank Test). In addition, as described above, the difference seen in the mass spectra between tumor and healthy specimens was correlated to radiochemical staining of tissue sections prepared from the same specimens, when the sections were treated with C-14 labelled uridine diphosphogalactose and bovine milk β 1,4-galactosyltransferase in galactosyltransferase reactions that were designed to quantitate non-reducing terminal N-acetylglucosamine residues in the sample glycoproteins. Moreover, the difference in radiochemical staining between the tumor and healthy control samples was the greatest when the respective difference in the degrees of galactosylation was the greatest, which allows for correlating the total amount of galactosyltransferase-sensitive non-reducing terminal β-N-acetylglucosamine residues in the tumor, with the mass spectrometric results obtained with isolated glycans from the tissue specimen.

*Individual lung carcinoma, a gastro-intestinal carcinoma, and kidney* hypernephroma *patients show severely decreased non-reducing terminal galactosylation of their glycoproteins.*

In addition to the general decrease in the galactosylation degree in the lung cancer tumor samples, the analyzed samples from the patient group included individuals in which the phenomenon was very drastic. In these samples, the N-glycan peak at m/z 1485.5 was either the most abundant or the second most abundant neutral glycan signal in the mass spectra, indicating that the corresponding glycan structure had been specifically accumulated in the tumor cell glycoproteins. A similar phenomenon was also detected in tumor samples from individual patients of kidney carcinoma and a gastro-intestinal carcinoma, indicating that it is not restricted to lung carcinoma. The accumulation of non-reducing terminal β-N-acetylglucosamine containing N-glycans also characterized the isolated tumor protein-linked glycans from a patient with kidney hypernephroma, which had previously been analyzed for its glycolipid structures and found to contain also glycolipids characterized by decreased galactosylation. The latter correlation between glycoprotein and glycolipid structures further suggests that our observed accumulation of non-reducing terminal β-N-acetylglucosamine containing glycoconjugates is a result of a general defect in galactosylation that occurs in various forms of cancer.

*Tumor sample from an individual lung carcinoma patient that shows severely decreased non-reducing terminal galactosylation of O- and N-linked glycoprotein glycans.*

Tumor sample from a non-small cell lung adenocarcinoma patient was studied in detail to elucidate the structures of various N-acetylglucosaminidase sensitive structures detected in the sample. Compared to healthy lung specimens, the mass spectrum of the non-sialylated glycan fraction from the paraffin-embedded and formalin-fixed tumor sample showed a significantly increased amount of a signal at m/z 609.23 (for $Hex_1HexNAc_2$, calc. m/z 609.21 for the ion $[M+Na]^+$) that can be assigned to a specific mucin-type O-glycan with specific exoglycosidase digestion reactions and MALDI-TOF mass spectrometry of the reaction products, as described below (mass spectra not shown). The abovementioned glycan signal was not sensitive to Jack bean α-mannosidase digestion, whereas signals corresponding to high-mannose type N-glycans with molecular formulae $Man_{3-8}GlcNAC_2$ and $Man_{2-3}GlcNAc_2Fuc_1$ present in the same sample were transformed into peaks at m/z 609.16 for $Hex_1HexNAc_2$ and m/z 755.22 for $Hex_1HexNAc_2Fuc_1$ (calc. m/z 755.27), respectively. Similarly, β-mannosidase digestion had no effect on the specific peak. However, β-N-acetylglucosaminidase from *S. pneumoniae* did cleave the peak at m/z 609.23 that was transformed into a peak at m/z 405.77. corresponding to $Hex_1HexNAc_1$ (calc. m/z 406.13), which indicates for the presence of a non-reducing terminal β-N-acetylglucosamine residue in the glycan structure. In addition, recombinant β1,3-galactosidase partly transformed the peak into a peak at m/z 447.16, $HexNAc_2$ (calc. m/z 447.16). The present data indicates that a major component glycan in the peak at m/z 609.23 contains both non-reducing terminal β-N-acetylglucosamine and non-reducing terminal β1,3-linked galactose residues, and indicates that it contains the O-glycan Core 2 trisaccharide structure GlcNAcβ1-6(Galβ1-3)GalNAc. Compared to healthy lung tissue specimens, the mass spectrum of the sialylated glycan fraction from the same tumor contained a significantly increased amount of a signal that can be assigned to a sialylated counterpart of the neutral trisaccharide described above, namely at m/z 876.27 for $NeuAc_1Hex_1HexNAc_2$ (calc. m/z 876.31 for the ion $[M-H]^-$). The data indicates that this glycan signal corresponds to the sialylated form of the Core 2 O-glycan epitope detected among the neutral glycans from the same specimen, i.e. GlcNAcβ1-6(Neu5Acα2-3Galβ1-3)Gal-NAc.

By the action *of S. pneumoniae* β-N-acetylglucosaminidase, the peak at m/z 1485.61 in the non-sialylated glycan fraction of the same sample, corresponding to $Hex_3HexNAc_4Fuc_1$ (calc. m/z 1485.53), was transformed into the peak at m/z 1079.16, corresponding to $Hex_3HexNAc_2Fuc_1$ (calc. m/z 1079.38), and the peak at m/z 1647.67, corresponding to $Hex_4HexNAc_4Fuc_1$ (calc. m/z 1647.59), was transformed into the peak at m/z 1444.24, corresponding to $Hex_4HexNAc_3Fuc_1$ (calc. m/z 1444.51), which indicates that the corresponding glycans contain 2 and 1 non-reducing terminal β-N-acetylglucosamine residues, respectively. By the combined action *of S. pneumoniae* β1,4-galactosidase and recombinant β1,3-galactosidase, both the peaks at m/z 1647.67 and m/z 1809.72, the latter corresponding to $Hex_5HexNAc_4Fuc_1$ (calc. m/z 1809.64), were converted into the peak at m/z 1485.48, which indicates that the corresponding glycans contained non-reducing terminal β-galactose residues in N-acetyllactosamine terminal structures. Taken together, the glycan signals at m/z 1485.61, 1647.67, and 1809.72 represent a series of differently galactosylated glycan species. The galactosylation degree calculated from these three signals in the present tumor sample is 24 %, which is significantly below the average 33 % of the tumor samples from the group of 7 lung cancer patients described above. Furthermore, the simultaneous presence of the $Hex_1HexNAc_2$ and $NeuAc_1Hex_1HexNAc_2$ terminal β-N-acetylglucosamine residue-containing glycans in the present tumor sample indicates that there is a general decrease in galactosylation of non-reducing terminal β-N-acetylglucosamine residues in the protein-linked glycans of the particular tumor sample.

Example 13 (reference). Protein and water solution-compatible conjugation reagents

*Preparation of UDP-GalN-PEG-fluorescein.*

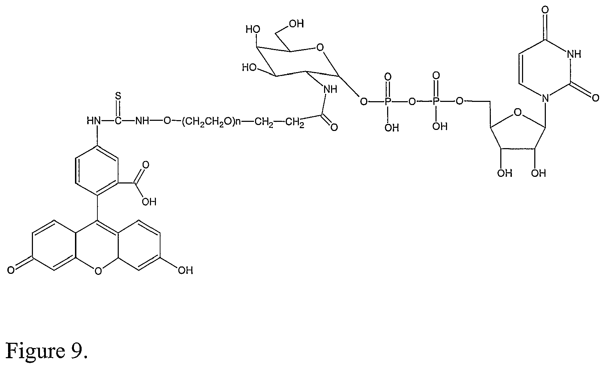

In 100 μl total reaction volume, 5 nmol uridine diphosphogalactosamine (UDP-GalN), 500 nmol fluorescein-poly(ethylene glycol)-NHS (MW 5000, Nektar Therapeutics, USA), and 500 nmol O-Benzotriazol-1-yl-N,N,N',N'-bis(tetramethylene)uronium hexafluorophosphate (Aldrich, USA), were incubated in ethylene glycol - dimethylformamide (1:1, v/v) containing 55 mM N-ethyldiisopropylamine, at room temperature for 60 hours. The reaction mixture was gel filtrated in a Superdex Peptide HR 10/30 column (Amersham Pharmacia Biotech) at 1 ml/min flow rate in 250 mM $NH_4HCO_3$ and the effluent was monitored with the UV detector at 214 nm, 261 nm, and 460 nm. Material eluted between 8 and 10 minutes, coeluting with the fluorescein-PEG starting material, was collected and dried. Anion exchange chromatography was performed on a Resource Q 1 ml column (Amersham Pharmacia Biotech) using $H_2O$-NaCl gradient. UV absorption at 214 nm, 261 nm, and 460 nm was monitored. Unbound material that contained the fluorescein-PEG starting material was discarded and the material eluting between 10 and 40 mM NaCl concentrations was collected. The fractions were analyzed by MALDI-TOF MS with a Voyager-DE STR BioSpectrometry Workstation in positive ion delayed extraction linear mode using 2,5-Dihydroxybenzoic acid (DHB) as the matrix. Mass spectrometry revealed that polyethylene glycol derivatives were presented in both fractions. Also UV absorption at 460 nm indicated that fluorescein was present in the both fractions. Taken together, the synthetized UDP-GalN-PEG-fluorescein (Fig. 9) was efficiently purified during the two chromatography steps.

*Enzyme reactions.*

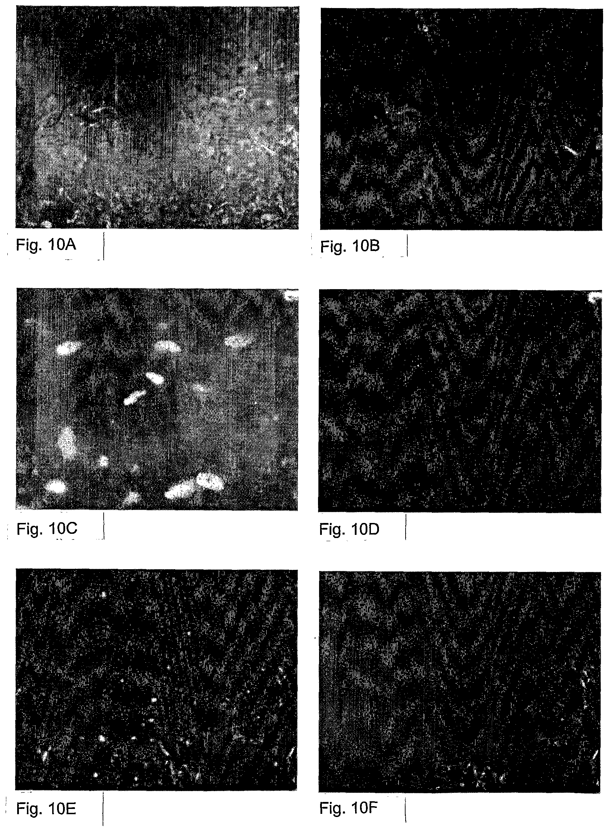

Formalin-fixed and paraffin-embedded tissue sections from human non-small cell lung adenocarcinoma tumor tissue, were deparaffinised and covered with incubation mixture containing UDP-GalN-PEG-fluorescein as a sugar donor, 20 mM $MnCl_2$, 10 mM Tris-HCl pH 8.0, and a mutant galactosyltransferase enzyme similar to the one described by Ramakrishnan and Qasba (2002). A negative control reaction contained the same incubation mixture without the enzyme. Reactions were incubated at 37 °C for 20 hours, after which the tissue sections were washed with water. The fluorescent samples were analyzed by Olympus AX70 Provis fluorescence microscope. After the reaction, the samples showed clear positive cells at wavelengths 460-490 nm (FITC) within the tumor tissue (Fig. 10.E). The fluorescence was in the cytoplasm as well as in the membrane on the positive cells (Fig. 10.C). However, the control samples remained negative (Fig. 10.A) showing only some autofluorescence (Fig. 10.B). However, the samples analyzed at wavelengths 530-550 nm were negative, indicating that the positive results were not due to autofluorescence (Fig. 10.D, 10.F). In conclusion, this example described how the enzyme catalysed the *ex vivo* transfer of GalN-PEG-fluorescein groups from UDP-GalN-PEG-fluorescein to non-reducing terminal N-acetylglucosamine (GlcNAc) residues present in glycoprotein glycans of human tumor cells and tumor tissue sections.

*Preparation and use of various conjugation reagents*

In different reactions, carboxylic acid reagents that were successfully incorporated to the 2-amino group of uridine diphosphogalactosamine through amidation, included S-acetyl-3-mercaptopropionic acid, 4-mercaptobutyric acid, Boc-2-aminooxyacetic acid, and N-maleimido-6-aminohexanoic acid, which are suitable for protein-compatible water-solution coupling of N-maleimido, aldehyde, and thiol group containing reagents or biologically active substances, respectively. The 3-mercaptopropionic acid conjugate was primarily produced as acetate protected form and the 2-aminooxyacetic acid conjugate as t-Boc protected form, and the protective groups can be completely removed by incubation in mild alkaline aqueous solution or trifluoroacetic acid, respectively. After purification, the structures of the conjugation reagents were confirmed by MALDI-TOF mass spectrometry and found to contain the desired functional groups attached to the galactosamine residue. In test reactions, the reagents were incubated in aqueous solution with a non-reducing terminal N-acetylglucosamine containing glycoconjugate and a modified galactosyltransferase enzyme similar to the one described by Ramakrishnan and Qasba (2002), which resulted in the successful transfer of conjugation reagent-modified galactosamine residues to the glycoconjugates, evidenced by MALDI-TOF mass spectrometry as a mass increase of the acceptor glycoconjugate, equivalent to the galactosamine-reagent conjugate (mass spectra not shown).

Example 14 (reference). Increased levels of specific antibodies against non-reducing terminal N-acetylglucosamine containing glycoconjugates in serum of cancer patients.

A group of four people with various stages of diagnosed cancer of the gastrointestinal tract or the ovary, was studied by analyzing serum samples for their concentrations of IgG and IgM antibodies against specific carbohydrate epitopes. The assay consisted of binding of specific antibodies from serum to synthetic glycoconjugates, washing, and then detecting the specifically bound antibody levels in ELISA with enzyme-labelled anti-human IgG or anti-human IgM secondary antibodies and quantitation of specific reaction products. The obtained results are depicted in Table 2. Both IgG and IgM levels against a non-reducing terminal N-acetylglucosamine containing N-glycan were elevated in one patient. Antibody levels against the Core 2 trisaccharide GlcNAcβ1-6(Galβ1-3)GalNAc were elevated in the whole patient group, but the specific antibody response varied: in one patient both IgG and IgM levels were elevated, in one patient mainly IgG, and in two patients mainly IgM. Antibody response against the GlcNAcβ1-3GalNAc disaccharide showed an identical pattern of antibody type response in the patient group. Two patients showed elevated serum levels of mainly IgM and one patient mainly IgG antibodies against an O-glycosidic β-GlcNAc epitope.

**Table 2.** Specific antibody responses against carbohydrate epitopes expressed as antibody signal intensities from which the assay background has been subtracted. Saccharide 1: GlcNAcβ1 -2Manα1 -6(GlcNAcβ1 -2Manα1 -3) Manβ1-4GlcNAcβ1 -4(Fucα1 -6)GlcNAcβ-N-Asn-R, Saccharide 2: positive cancer-associated saccharide, Saccharide 3: GlcNAcβ1-6(Galβ1-3)GalNAcα-O-CH$_2$-R, Saccharide 4: GlcNAcβ-O-CH$_2$-R, Saccharide 5: GlcNAcβ1-3GalNAcα-O-CH$_2$-R. R: variable linker.

| Saccharide | 1 | 2 | 3 | 4 | 5 |
|---|---|---|---|---|---|
| Patient 1 IgG | 0 | 0 | 0,039 | 0 | 0,017 |
| Patient 1 IgM | 0 | 0,259 | 0,091 | 0,159 | 0,125 |
| Patient 2 IgG | 0 | 0,022 | 0,078 | 0,082 | 0,094 |
| Patient 2 IgM | 0 | 0,079 | 0,008 | 0,029 | 0,041 |
| Patient 3 IgG | 0,006 | 0 | 0 | 0,013 | 0 |
| Patient 3 IgM | 0,01 | 0,1 | 0,068 | 0,122 | 0,011 |
| Patient 4 IgG | 0 | 0 | 0 | 0 | 0 |
| Patient 4 IgM | 0 | 0,463 | 0,042 | 0 | 0,056 |

Example 15 (reference).

Activity of β1,4-galactosyltransferase in human serum and activation of added β1,4-galactosysyltransferase under various reaction conditions

Fresh human blood sample was collected from blood group B -individual (5 ml). After clotting for 30 min it was centrifuged at + 4°C, 2000 rpm for 25 minutes, and the serum was collected for experiments (2 ml, stored on ice).

Reactions were performed with 50 nmol of acceptor saccharide (GlcNAcβ1-3Galβ1-4GlcNAc, from enzymatic synthesis, purified by ion exhange and gel filtration, characterized by NMR and mass spectrometry), 5 nmol UDP-[$^{14}$C]Gal (mixture of nonlabelled and radioactive, radioactivity was 100 000 cpm) and 20 μl of fresh human serum (total volume was 20 μl). Reaction conditions were varied by adding cations and bovine milk β1,4-galactosyltransferase (β1,4GalT).

Results

By adding 2 mU of β1,4GalT alone showed clear galactosyltransferase activity, purified galactosylated tetrasaccharide sample had 534 cpm (Table 3). The backround is likely radioactive Gal released from donor substrate. Adding 0.1 mM Zn$^{2+}$ to reaction mixture doubled the product formation. Further addition of 4 mM MgCl$_2$, and 2 mM CaCl$_2$ increased additionally the amount of product.

Similar increasing in product formation was with Mn$^{2+}$ -addition, but without external β1,4GalT, other salts used in the experiment did not activate the endogenous galactosyltransferase activity of human serum. Also MnCl$_2$ at lower concentration than 5 mM was able to activate the endogenous enzyme in the biological solution (not shown). Notable is also that background was lower with Mn$^{2+}$ and Zn$^{2+}$ together with fosforylcholine. Fosforylcholine may inhibit enzyme activities of serum which degradate the donor nucleotide.

Table 3

| Changes in reaction mixture | Product (cpm) | Background (cpm) |
|---|---|---|
| No salt, 2 mU β1,4GalT | 534 | 5104 |
| 0.1 mM ZnCl$_2$, 2 mU β1,4GalT | 1022 | 4446 |
| 0.1 mM ZnCl$_2$, 2 mU β1,4GalT, 4 mM MgCl$_2$, 2 mM CaCl$_2$ | 1292 | 4546 |
| 0.1 mM ZnCl$_2$, 1 mM fosforylcholine 2 mU β1,4GalT | 872 | 2552 |
| 5 mM MnCl$_2$ | 566 | 1079 |
| 1 mM MnCl$_2$ | 1073 | 2068 |
| 0.2 mM MnCl$_2$ | 457 | 4578 |

Example 16 (reference).

a) The oligosaccharide structure GlcNAcβ3Galβ4GlcNAcβ3Galβ4Glc is receptor for the gastric pathogen *Helicobacter pylori.* The structure is changed to 10-fold weaker receptor structure Galβ4GlcNAcβ3Galβ4GlcNAcβ3Galβ4Glc by incubating it with UDP-Gal and β4-galactosyltransferase from bovine milk.

b) The oligosaccharide receptor GlcNAcβ3Galβ4GlcNAcβ3Galβ4Glc is incubated with GDP-Fuc and soluble human fucosyltransferase VI corresponding to major the fucosyltransferase from human milk. MALDI-TOF mass spectrometric analysis reveled a major peak corresponding to GlcNAcβ3Galβ4(Fucα3)GlcNAcβ3Galβ4Glc, the structure is confimed by NMR-spectroscopy.

c) Erythrocytes from a patient suffering from glycosylation deficiency causing high levels of terminal GlcNAc-structures including GlcNAcβ3Galβ4GlcβCer are incubated with radiolabeled UDP-Gal and βgalactosyltransferase. Transfer of labeled galactose on the cell surface is observed. The galactosylation of the cells reduces the reactivity of the cells with anti-GlcNAc-antibodies.

**References**

Ångstrom, J., and Karl-Anders Karlsson (1996) Glycobiology 6, 599-609.

Arap, W., Pasqualini, R. and Ruoslahti, E. (1998) Science 279, 323-4.

Endo et al. (1996) Eur. J. Biochem 236:579-590.

Ernst, B., Hart, G.W., and Sinaÿ, P. (eds.) (2000) Crbohydrates in chemistry and biology, ISBN 3-527-29511-9, Weiley-VHC, Weinheim.

Hanisch, F.-G., Koldovsky, U., and Borchard F. (1993) Cancer Res. 53, 4791-4796.

Hansson, G.C., Karlsson, K.-A., Larson G., Stromberg, N., and Thurin, J. (1985) Anal. Biochem. 146, 158-63.

Harvey, D.J., et al. (1993) Rapid Commun. Mass Spectrom. 7(7):614-9.

Hounsell, E.F., Lawson, A.M., Stoll, M., Kane, D.P., Cashmore, G.C., Carruthers, R.A., Feeney, J., and Feizi, T. (1989) Eur. J. Biochem. 186, 597-610.

Holmes, E.H., and Greene, T.G. (1991) Arch. Biochem. Biophys. 288, 87-96.

Hu, J., Stults, C.L.M., Holmes, E.H., and Macher, B.A. (1994) Glycobiology 4, 251-257.

Nakamura, M., Tsunoda, A., Sakoe, K., and Saito, M. (1993) Biochem. Biophys. Res. Commun. 197 1025-1033.

Manzi, A.E., et al. (2000) Glycobiology 10(7):669-89.

Meichenin M. et al. (2000) Cancer Research 60:5499-5507.

Mäki, M., et al. (2002) Eur. J. Biochem. 269(2):593-601.

Nyman, T.A., et al. (1998) Eur. J. Biochem. 253(2):485-93.

Packer, N.H., et al. (1998) Glycoconj. J. 15(8):737-747.

Ramakrishnan, B., and Qasba, P.K. (2002) J. Biol. Chem. 277(23):20833-9.

Papac, D.I., et al. (1996) Anal. Chem. 68(18):3215-23.

Rhen M., Klemm P., and Korhonen T.K. (1986). J Bacteriol 168, 1234-42 .

Saarinen, J., et al. (1999) Eur. J. Biochem. 259(3):829-40.

Sadamoto, R., Nikura, K., Nishimura, S.-I. (2001) Chemical engineering of bacterial cell wall. Poster C13.8, XVI International Symposium on Glycoconjugates August 19.-24. 2001 Haag Netherlands, Glycoconjugate J. no. 1 /2001.

Spillmann, A., and Finne, J. (1994) Eur. J. Biochem. 220, 385-394.

Symington, F.W, Hendersson, B.A., and Hakomori, S.-I. (1984) Mol. Immunol. 21, 877-882.

Teneberg, S., Lönnroth, I., Torres Lopez, J.T., Galili, U., Ölwegård Halvarsson, M., Verostek, M.F., et al. (2000) Anal. Biochem. 278:111-122.

Teneberg S, Angstrom J, Jovall P-Å, and Karlsson K-A. (1994) J Biol Chem 269, 8554-63

Viitala, J., and Finne, J. (1984) Eur. J. Biochem. 138, 393-397.

**Claims**

1.  Use of a composition comprising

i) a 2-modified monosaccharide derivative according to the formula

Nu-HexL-S-T

wherein Hex is Gal or GalNAc, Nu is UDP,
L is a linking atom on carbon 2 of the Gal or GalNAc, S is a spacer group, atom or nothing, and T is a label or a chemoselective and protein/tissue compatible linking group; and

ii) a galactosyltransferase which is engineered to transfer effectively a 2-modified monosaccharide derivative glycosidically linked to a nucleotide residue; in the in vitro diagnostics of cancer or tumor.

2. The use according to claim 1, wherein L is an oxygen, nitrogen, carbon or sulphur atom.

**Patentansprüche**

1. Verwendung einer Zusammensetzung, umfassend

i) ein 2-modifiziertes Monosacchariddderivat nach der Formel

Nu-HexL-S-T

wobei Hex Gal oder GalNAc ist, Nu UDP ist,
L ein Bindungsatom auf Kohlenstoff 2 des Gal oder GalNAc ist, S eine Spacer-Gruppe, ein Atom oder nichts ist, und T eine Bezeichnung oder eine chemoselektive und/ oder protein-/gewebekompatible Bindegruppe ist; und

ii) eine Galactosyl-Transferase, die hergestellt ist, um ein an einen Nukleotidrest glycosidisch gebundenes 2-modifiziertes Monosacchariddderivat effektiv zu übertragen;

in der In-vitro-Diagnose von einem Krebs oder einem Tumor.

2. Verwendung nach Anspruch 1, wobei L ein Sauerstoff-, Stickstoff-, Kohlenstoff-, oder Schwefelatom ist.

**Revendications**

1. Utilisation d'une composition comprenant

i) un dérivé de monosaccharide modifié en 2 selon la formule

Nu-HexL-S-T

dans laquelle Hex est Gal ou GalNAc, Nu est UDP,
L est un atome de liaison sur le carbone 2 de Gal ou GalNAc, S est un groupe espaceur, un atome ou rien, et T est un marqueur ou un groupe de liaison chimiosélectif et compatible avec un tissu/une protéine ; et

ii) une galactosyltransférase qui est conçue pour transférer efficacement un dérivé de monosaccharide modifié en 2 lié de manière glycosidique à un résidu de nucléotide ; dans le diagnostic in vitro d'un cancer ou d'une tumeur.

2. Utilisation selon la revendication 1, dans laquelle L est un atome d'oxygène, d'azote, de carbone ou de soufre.

No of Sugars

5 ~

7 ~

10-15

Tumor   Normal
tissue   tissue

Autoradiogram after overlayering with
$^{35}$S-methionine- labeled E.coli
Specificity:    GlcNAcβ

# Figure 1.

A. G-fimbriated *E. coli*    B. *E. cristagalli* lectin

Figure 2.

Figure 3A.

EP 1 534 324 B1

Figure 3B.

EP 1 534 324 B1

Figure 3C.

EP 1 534 324 B1

Figure 3D.

Figure 4A.

EP 1 534 324 B1

Figure 4B.

36

Figure 5.

a.          b.

Figure 6.

Figure 7A.

Figure 7B.

Figure 7C.

Figure 8.

Figure 9.

Fig. 10A

Fig. 10B

Fig. 10C

Fig. 10D

Fig. 10E

Fig. 10F

## REFERENCES CITED IN THE DESCRIPTION

*This list of references cited by the applicant is for the reader's convenience only. It does not form part of the European patent document. Even though great care has been taken in compiling the references, errors or omissions cannot be excluded and the EPO disclaims all liability in this regard.*

### Patent documents cited in the description

- US 5102663 A
- US 5660834 A
- US 5747048 A
- US 5229289 A
- US 6083929 A
- US 4851511 A
- US 4904596 A
- US 5874060 A
- US 6025481 A
- US 5795961 A
- US 4725557 A
- US 5059520 A
- US 5171667 A
- US 5173292 A
- US 6090789 A
- US 5708163 A
- US 5679769 A
- US 5543505 A
- US 5902725 A
- US 6203999 B
- DE 3807594 A1
- WO 0021552 A
- FI 20011671
- WO 03094842 A
- US 4725 A
- US 557 A

### Non-patent literature cited in the description

- **MOCZAR et al.** *FEBS Letters,* 1975, vol. 50 (3), 300-302
- **ZENG et al.** *Analytical Biochemistry,* 1996, vol. 239 (1), 99-106
- **BÜLTER et al.** *Chembiochem - A European Journal of Chemical Biology,* 2001, vol. 2, 884-894
- *Carbohydr. Res.,* 1998, vol. 322, 167
- *Carbohydr. Res.,* 1997, vol. 297, 1
- *Eur. J. Biochem.,* 1998, vol. 257, 29
- **ÅNGSTROM, J. ; KARL-ANDERS KARLSSON.** *Glycobiology,* 1996, vol. 6, 599-609
- **ARAP, W. ; PASQUALINI, R. ; RUOSLAHTI, E.** *Science,* 1998, vol. 279, 323-4
- **ENDO et al.** *Eur. J. Biochem,* 1996, vol. 236, 579-590
- Crbohydrates in chemistry and biology. Weiley-VHC, 2000
- **HANISCH, F.-G. ; KOLDOVSKY, U. ; BORCHARD F.** *Cancer Res.,* 1993, vol. 53, 4791-4796
- **HANSSON, G.C. ; KARLSSON, K.-A. ; LARSON G. ; STROMBERG, N. ; THURIN, J.** *Anal. Biochem.,* 1985, vol. 146, 158-63
- **HARVEY, D.J. et al.** *Rapid Commun. Mass Spectrom.,* 1993, vol. 7 (7), 614-9
- **HOUNSELL, E.F. ; LAWSON, A.M. ; STOLL, M. ; KANE, D.P. ; CASHMORE, G.C. ; CARRUTHERS, R.A. ; FEENEY, J. ; FEIZI, T.** *Eur. J. Biochem.,* 1989, vol. 186, 597-610
- **HOLMES, E.H. ; GREENE, T.G.** *Arch. Biochem. Biophys.,* 1991, vol. 288, 87-96
- **HU, J. ; STULTS, C.L.M. ; HOLMES, E.H. ; MACHER, B.A.** *Glycobiology,* 1994, vol. 4, 251-257
- **NAKAMURA, M. ; TSUNODA, A. ; SAKOE, K. ; SAITO, M.** *Biochem. Biophys. Res. Commun.,* 1993, vol. 197, 1025-1033
- **MANZI, A.E. et al.** *Glycobiology,* 2000, vol. 10 (7), 669-89
- **MEICHENIN M. et al.** *Cancer Research,* 2000, vol. 60, 5499-5507
- **MÄKI, M. et al.** *Eur. J. Biochem.,* 2002, vol. 269 (2), 593-601
- **NYMAN, T.A. et al.** *Eur. J. Biochem.,* 1998, vol. 253 (2), 485-93
- **PACKER, N.H. et al.** *Glycoconj. J.,* 1998, vol. 15 (8), 737-747
- **RAMAKRISHNAN, B. ; QASBA, P.K.** *J. Biol. Chem.,* 2002, vol. 277 (23), 20833-9
- **PAPAC, D.I. et al.** *Anal. Chem.,* 1996, vol. 68 (18), 3215-23
- **RHEN M. ; KLEMM P. ; KORHONEN T.K.** *J Bacteriol,* 1986, vol. 168, 1234-42
- **SAARINEN, J. et al.** *Eur. J. Biochem.,* 1999, vol. 259 (3), 829-40
- **SADAMOTO, R. ; NIKURA, K. ; NISHIMURA, S.-I.** Chemical engineering of bacterial cell wall. Poster C13.8, XVI International Symposium on Glycoconjugates. *Glycoconjugate J.,* 2001
- **SPILLMANN, A. ; FINNE, J.** *Eur. J. Biochem.,* 1994, vol. 220, 385-394
- **SYMINGTON, F.W ; HENDERSSON, B.A. ; HAKOMORI, S.-I.** *Mol. Immunol.,* 1984, vol. 21, 877-882

- **TENEBERG, S. ; LÖNNROTH, I. ; TORRES LOPEZ, J.T. ; GALILI, U. ; ÖLWEGÅRD HALVAR-SSON, M. ; VEROSTEK, M.F. et al.** *Anal. Biochem.,* 2000, vol. 278, 111-122
- **TENEBERG S ; ANGSTROM J ; JOVALL P-Å ; KARLSSON K-A.** *J Biol Chem,* 1994, vol. 269, 8554-63
- **VIITALA, J. ; FINNE, J.** *Eur. J. Biochem.,* 1984, vol. 138, 393-397